# EUROPEAN PATENT APPLICATION

(11) **EP 4 763 999 A1**
(43) Date of publication of application: **24.06.2026**
(21) Application number: 24383394.4
(22) Date of filing: 18.12.2024
(51) Int. Cl.: C12Q 1/6886

(54) **METHODS AND REAGENTS FOR DINSTINGUISHING BETWEEN UTERINE TUMORS**

(71) Applicant: IGENOMIX S.L., 46980 Valencia (ES)
(72) Inventor: AMADOZ, Alicia, E-46980 Paterna, Valencia (ES); JIMENEZ ALMAZAN, Jorge, E-46980 Paterna, Valencia (ES); MÁS PERUCHO, Aymara, E-46980 Paterna, Valencia (ES)
(74) Representative: ABG Intellectual Property Law, S.L.

(57) **Abstract**

The present invention relates to methods for distinguishing between leiomyosarcoma and leiomyoma, as well as to a method for identifying a subject suspected of having leiomyosarcoma. The invention also relates to kits and devices for carrying out said methods of the invention.

## Description

### FIELD OF THE INVENTION

The present invention relates to the field of gynecological cancer diagnostics and, more in particular, to methods for distinguishing between uterine leiomyosarcoma and uterine leiomyoma in a subject, as well as to a method for identifying a subject suspected of having uterine leiomyosarcoma, and to kits and devices for carrying out said methods.

### BACKGROUND OF THE INVENTION

Uterine leiomyomas (LM) are benign tumors arising in the smooth muscle cells of the uterine wall. They are the most common pelvic tumors in women, with a prevalence of >80% for African American and -70% for Caucasian women before 50 years of age. Although LM are non-malignant tumors, the risk of hidden undiagnosed malignancy, such as uterine leiomyosarcoma (LMS), occurs in one among 498 uterine tumors.

Laparoscopic myomectomy with morcellation of the tumor is the gold standard therapeutic option for uterine tumors. Unfortunately, clinical symptoms as well as morphological features between LM and LMS are indistinguishable prior to surgery introducing the risk of potential spread of undiagnosed LMS causing early metastasis, poor prognosis and high recurrence rates with limited therapeutic efficacy when the treatment of LMS is by surgery. For this reason, the FDA issued a press release in 2014 discouraging the use of power morcellators to treat myometrial tumors, substituting laparoscopic myomectomy for laparotomy-based procedures and thus increasing morbidity, mortality, and the cost for the patient and healthcare system. Therefore, the development of accurate and non-invasive diagnostic methods is a priority in the field of gynecology and oncology, especially in the health improvement of patients with surgical indication for hysterectomy, laparoscopic or laparotomic myomectomy for diagnosis of uterine tumors. Recently, the concept of "liquid biopsy" has emerged as a minimally invasive alternative to surgical biopsies for solid tumors with highly recurrent mutations, avoiding the sampling of tumor tissue before and after treatment.

Additionally, the absence of standardized preoperative biomarkers to differentiate uterine LMS versus LM represents an important diagnostic challenge. Thus, the search for standardized molecular criteria to differentiate uterine LMS and LM before surgery to prevent dissemination of hidden malignancies during morcellation represents an important current diagnostic challenge. In fact, available "omics" profiling for LMS has been limited due to the rare incidence of this malignancy (1 in 498 patients undergoing hysterectomy or myomectomy for presumed LM).

Given these challenges, there is an urgent need to develop more effective strategies for preoperative differential molecular diagnosis of uterine tumors.

WO2023061914 discloses methods for the differential diagnosis of LMS and LM, as well as methods for the prognosis of patients suffering from LMS which are based on transcriptomic analysis, genomic coverage analysis, genomic mutational analysis (determination of SNV (single nucleotide variant) biomarkers), and the determination of CNVs (copy number variants) in a biopsy sample.

### SUMMARY OF THE INVENTION

The authors of the present invention, using whole-exome and RNA sequencing (RNAseq), have identified the differential molecular footprint of LMS versus LM. In particular, the authors of the invention have found different gene expression levels between LMS and LM based on the specific transcriptomic profile detected at the RNA level. Leveraging this data, differential gene expression analysis was performed to assess the effect of the differentially expressed genes on gene expression across the study population.

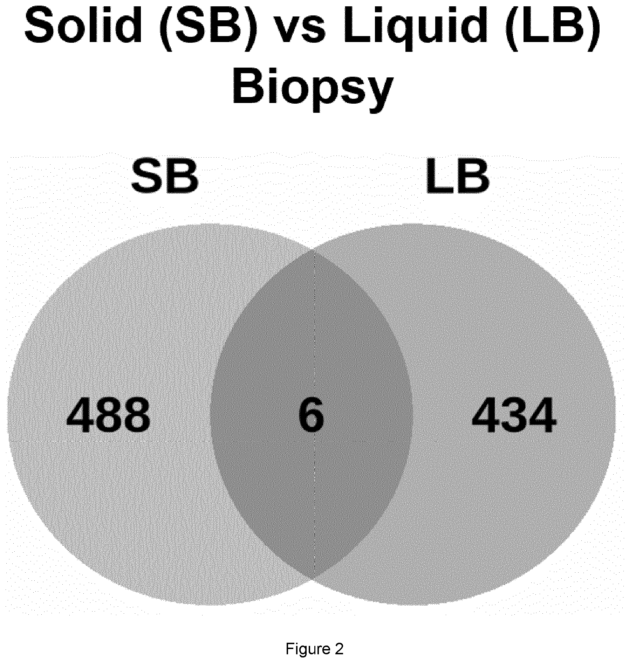

Therefore, a first aspect of the invention pertains to an *in vitro* method for distinguishing between uterine leiomyosarcoma or uterine leiomyoma in a subject suspected of having one of these conditions, the method comprising:
(i) measuring the level of expression of at least one gene selected from POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene in a biological sample obtained from the subject, and
(ii) comparing said level of expression with a reference value,
wherein a deviation in the level of expression of said at least one gene with respect to said reference value is indicative that the subject is affected by uterine leiomyosarcoma.

In a second aspect, the invention pertains to an *in vitro* method for distinguishing between uterine leiomyosarcoma or uterine leiomyoma in a subject suspected of having one of these conditions, the method comprising:
(i) measuring the level of expression of at least one gene selected from the list shown in Table 2, the list shown in Table 3, the list shown in Table 4, the list shown in Table 6 or the list shown in Table 7 in a biological sample obtained from the subject, and
(ii) comparing said level of expression with a reference value,
wherein a deviation in the level of expression of said at least one gene with respect to said reference value is indicative that the subject is affected by uterine leiomyosarcoma.

In a third aspect, the invention pertains to an *in vitro* method for identifying a subject suspected of having uterine leiomyosarcoma as a candidate to receive a suitable therapy to treat uterine leiomyosarcoma, the method comprising:
(i) determining whether the subject is affected by uterine leiomyosarcoma following any of the methods according to the first or the second aspects of the invention; and
(ii) designating said subject as a candidate to receive a suitable therapy to treat uterine leiomyosarcoma if the subject is diagnosed as having uterine leiomyosarcoma.

In another aspect, the invention pertains to a method for treating uterine leiomyosarcoma in a subject in need thereof, comprising the administration of a therapy suitable for the treatment of uterine leiomyosarcoma, wherein the subject to be treated has been identified using any of the methods according to the first or the second aspects of the invention.

In yet another aspect, the invention pertains to a chemotherapeutic agent, hormonal agent and/or targeted agent for use in the treatment of uterine leiomyosarcoma, wherein the subject to be treated has been identified through any of the methods according to the first or the second aspects of the invention.

In another aspect, the invention relates to a kit, package and/or device comprising reagents adequate for implementing any of the methods according to the first, the second or the third aspects of the invention.

In a further aspect, the invention pertains to a computer-implemented method, wherein the method is any of the methods according to the first, the second or the third aspects of the invention.

In a yet further aspect, the invention pertains to a computer comprising instructions for carrying out any of the methods according to the first, the second or the third aspects of the invention.

### BRIEF DESCRIPTION OF THE FIGURES

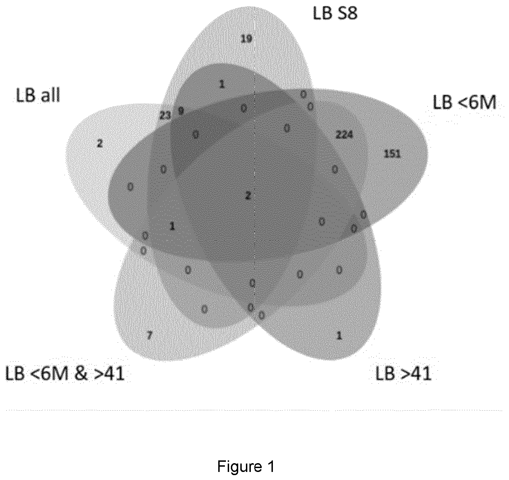

**Figure 1****:** Venn diagram displaying differentially expressed genes (DEGs) obtained using 5 selected comparisons between Leiomyosarcoma (LMS) and Leiomyoma (LM) liquid biopsy samples. "LB all" refers to the number of DEGs (37) obtained in the analysis using all samples; "LB S8" refers to the number of DEGs (55) obtained in the analysis using samples filtered by a quality threshold; "LB <6M" refers to the number of DEGs (378) obtained in the analysis using samples which have been frozen for less than 6 months; "LB >41" refers to the number of DEGs (13) obtained in the analysis using samples which derive from subjects over 41 years of age; and "LB <6M & >41" refers to the number of DEGs (234) obtained in the analysis using samples which have been frozen for less than 6 months and which derive from subjects over 41 years of age.
**Figure 2****:** DEGs in common between results from Solid (SB) and Liquid (LB) Biopsies of Leiomyosarcoma (LMS) and Leiomyoma (LM) samples.
**Figure 3****:** Volcano plot showing DEGs (diamond-shaped dots) between LMS and LM obtained from the analysis including all samples from the study. Genes with a |log2FC| is ≥ 2 are shown in cross-shaped dots. Genes with a non-significant result are shown in circle-shaped dots (NS).
**Figure 4****:** Volcano plot showing DEGs (diamond-shaped dots) between LMS and LM obtained from the analysis of the samples filtered by a quality threshold. Genes with a |log2FC| is ≥ 2 are shown in cross-shaped dots. Genes with a non-significant result are shown in circle-shaped dots (NS).
**Figure 5****:** Volcano plot showing DEGs (diamond-shaped dots) between LMS and LM obtained from the analysis of the samples which have been frozen for less than 6 months.
Genes with a |log2FC| is ≥ 2 are shown in cross-shaped dots. Genes with a non-significant result are shown in circle-shaped dots (NS).
**Figure 6****:** Volcano plot showing DEGs (diamond-shaped dots) between LMS and LM obtained from the analysis of the samples which derive from subjects over 41 years of age. Genes with a |log2FC| is ≥ 2 are shown in cross-shaped dots. Genes with a non-significant result are shown in circle-shaped dots (NS).
**Figure 7****:** Volcano plot showing DEGs (diamond-shaped dots) between LMS and LM obtained from the analysis of the samples which have been frozen for less than 6 months and which derive from subjects over 41 years of age. Genes with a |log2FC| is ≥ 2 are shown in cross-shaped dots. Genes with a non-significant result are shown in circle-shaped dots (NS).

### DETAILED DESCRIPTION OF THE INVENTION

### First method of the invention (method based on transcriptomic analysis)

The authors of the present invention have found that LM and LMS have specific transcriptomic profiles and have carried out a comparative transcriptomic analysis between histologically confirmed LM (n = 72) and LMS (n = 23) tumors. The results have revealed 13 genes that were differentially expressed obtained from 5 selected comparisons between LMS and LM liquid biopsy samples. Selected group comparisons differed in demographic and technical characteristics, such as the age of the subject, or the freezing time of the sample prior to the RNA extraction and data quality. This difference in the transcriptomic profiles allows distinguishing between one disease or the other in a subject by analyzing the RNA composition in a sample from the subject and classifying the subject affected by LMS or LM using a transcriptomic analysis-based method. Accordingly, in a first aspect, the invention pertains to an *in vitro* method for distinguishing between uterine leiomyosarcoma or uterine leiomyoma in a subject suspected of having one of these conditions (hereinafter referred to as "the first method of the invention"), the method comprising:
(i) measuring the level of expression of at least one gene selected from POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene in a biological sample obtained from the subject, and
(ii) comparing said level of expression with a reference value,
wherein a deviation in the level of expression of said at least one gene with respect to said reference value is indicative that the subject is affected by uterine leiomyosarcoma.

The expression "distinguishing between", as used herein, refers to the process of identifying which disease or condition is most likely causing a subject's symptoms when multiple diseases or conditions with similar symptoms are possible, or distinguishing of a particular disease or condition from others that present similar clinical features based on an analysis of the clinical data. This determination, as it is understood by a person skilled in the art, does not claim to be correct in 100% of the analyzed samples. However, it requires that a statistically significant amount of the analyzed samples is classified correctly. The amount that is statistically significant can be established by a person skilled in the art by means of using different statistical methods. Illustrative, non-limiting examples of said statistical methods include determining confidence intervals, determining the p-value, the Student's t-test or Fisher's discriminant functions, etc. (see, for example, Dowdy and Wearden, Statistics for Research, John Wiley & Sons, New York 1983). The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present invention preferably allow correctly diagnosing in at least 60%, in at least 70%, in at least 80%, or in at least 90% of the subjects of a determined group or population analyzed.

The term "uterine leiomyoma", also known as "uterine fibroid", as used herein, refers to a benign tumor that appears in the smooth muscular layer of the uterus.

The term "uterine leiomyosarcoma", as used herein, refers to a malignant tumor which originates in the smooth muscular layer of the uterus. The term includes both primary tumors as well as metastasis.

In a first step, the first method of the invention comprises the determination of the level of expression of at least one gene selected from POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene in a biological sample obtained from the subject whose diagnosis is to be determined. In some embodiments, the first step of the first method of the invention comprises the determination of the expression levels of at least 2, at least 3, at least 4, at least 5, at least 6, at least 7, at least 8, at least 9, at least 10, at least 11, at least 12 or the 13 genes selected from POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene.

The term "expression level", as used herein, refers to the measurable quantity of a gene product produced by the gene in a sample of the subject, wherein the gene product can be a transcriptional product or a translational product. As understood by the person skilled in the art, the gene expression level can be quantified by measuring the messenger RNA (mRNA) levels of said gene or of the protein encoded by said gene. In the context of the present invention, the expression level of the genes used in the methods according to the invention can be determined by measuring the levels of mRNA encoded by said gene, or by measuring the levels of the protein encoded by said gene, i.e. the protein or variants thereof. Variants of the proteins encoded by the genes which are measured according to the methods of the invention include all the physiologically relevant post-translational chemical modifications forms of the protein, for example, glycosylation, phosphorylation, acetylation, etc., provided that the functionality of the protein is maintained.

The term "sample" or "biological sample", as used herein, refers to biological material isolated from a subject. The biological sample contains any biological material suitable for detecting DNA, RNA or protein levels. In a particular embodiment, the sample comprises genetic material, e.g., DNA, genomic DNA (gDNA), complementary DNA (cDNA), RNA, heterogeneous nuclear RNA (hnRNA), mRNA, etc., from the subject under study. The sample can be isolated from any suitable tissue or biological fluid such as, for example blood, saliva, plasma, serum, urine, cerebrospinal liquid (CSF), feces, a surgical specimen, a specimen obtained from a biopsy, and a tissue sample embedded in paraffin. Methods for isolating samples are well known to those skilled in the art. In particular, methods for obtaining a sample from a biopsy include gross apportioning of a mass, or micro-dissection or other art-known cell-separation methods. In order to simplify conservation and handling of the samples, these can be formalin-fixed and paraffin-embedded or first frozen and then embedded in a cryosolidifiable medium, such as OCT-compound, through immersion in a highly cryogenic medium that allows rapid freeze.

Alternatively, the sample from the subject according to the methods of the present invention is a biological fluid sample. The term "biological fluid" and "biofluid" are used interchangeably herein and refer to aqueous fluids of biological origin.

The biofluid may be obtained from any location (such as blood, plasma, serum, urine, bile, cerebrospinal fluid, aqueous or vitreous humor, or any bodily secretion), an exudate (such as fluid obtained from an abscess or any other site of infection or inflammation), or fluid obtained from a joint (such as a normal joint or a joint affected by disease such as rheumatoid arthritis).

In a particular embodiment, the sample from the subject according to the methods of the present invention is selected from the group consisting of blood, plasma and serum, and a tissue sample; more preferably from the group consisting of plasma and a tissue sample.

The term "subject" or "patient" refers herein to a person in need of the analysis described herein. In some embodiments, the subject is a patient. In some embodiments, the subject is a human. In some embodiments, the subject is a female human (a woman). In some embodiments the subject is a female presenting with pathology and/or history consistent with uterine fibroids believed to be a benign neoplasm. In some embodiments the subject is a female presenting with pathology and/or history consistent with uterine fibroids believed to be leiomyoma (LM). In some embodiments the subject is a female presenting with pathology and/or history consistent with uterine fibroids believed to be leiomyoma and desiring surgical intervention. In some embodiments the subject is a female presenting with pathology and/or history consistent with uterine fibroids believed to be leiomyoma, desiring surgical intervention, and requiring an evaluation of the neoplasm to evaluate the risk that the neoplasm is malignant in order to guide the selection of therapy. In some embodiments the subject is a female presenting with pathology and/or history consistent with uterine fibroids, desiring surgical intervention and requiring an evaluation of the neoplasm to evaluate the risk that the neoplasm is a leiomyosarcoma in order to guide the selection of therapy.

In some embodiments, the sample wherein the expression level of the POC1A, PTPRT, PTCHD1, MUC4, CENPM, KLHL41, FLG2, GPR52, MB21D1, MT-ND1, MT-CO2, FAM21B or GSTM1 is determined can be any sample containing cells from the potential tumor. In a particular embodiment, the sample containing cells from the potential tumor is a potential tumor tissue or a portion thereof. In a more particular embodiment, said potential tumor tissue sample is a uterine tissue sample from a patient in which the method for distinguishing between uterine leiomyosarcoma or uterine leiomyoma in a subject suspected of having uterine leiomyosarcoma or uterine leiomyoma is to be carried out. Said sample can be obtained by conventional methods, e.g., biopsy, surgical excision, or aspiration, by using methods well known to those of ordinary skill in the related medical arts. Methods for obtaining the sample from the biopsy include gross apportioning of a mass, or microdissection or other art-known cell-separation methods including partial tumorectomy. Tumor cells can additionally be obtained from fine needle aspiration cytology. In some embodiments, the sample has been obtained by hysterectomy or laparoscopic/laparotomic myomectomy.

In order to simplify conservation and handling of the samples, these can be formalin-fixed and paraffin-embedded or first frozen and then embedded in a cryosolidifiable medium, such as OCT-compound, through immersion in a highly cryogenic medium that allows for rapid freeze.

In a particular embodiment of the first method of the invention, the sample wherein the expression levels of the POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene are determined, is a tumor sample obtained by hysterectomy or laparoscopic/laparotomic myomectomy.

In another particular embodiment, the sample containing the cells from the potential tumor is a biofluid. In a more particular embodiment, said biofluid is selected from the group consisting of blood, plasma and serum from a patient in which the method for distinguishing between uterine leiomyosarcoma or uterine leiomyoma in a subject suspected of having uterine leiomyosarcoma or uterine leiomyoma is to be carried out. Said biofluid sample can be obtained by conventional methods well known to those of ordinary skill in the related arts.

In yet another particular embodiment of the first method of the invention, the sample wherein the expression levels of the POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene are determined, is a biofluid obtained by any art-appropriate means.

Gene expression levels can be quantified by measuring the messenger RNA (mRNA) levels of the gene or of the protein encoded by said gene, i.e. the POC1A protein, PTPRT protein, PTCHD1 protein, MUC4 protein, CENPM protein, KLHL41 protein, FLG2 protein, GPR52 protein, MB21D1 protein, MT-ND1 protein, MT-CO2 protein, FAM21B protein or GSTM1 protein or of variants thereof. The POC1A protein, PTPRT protein, PTCHD1 protein, MUC4 protein, CENPM protein, KLHL41 protein, FLG2 protein, GPR52 protein, MB21D1 protein, MT-ND1 protein, MT-CO2 protein, FAM21B protein or GSTM1 protein variants include all the physiologically relevant post-translational chemical modifications forms of the protein, for example, glycosylation, phosphorylation, acetylation, etc., provided that the functionality of the protein is maintained. Said term encompasses the POC1A protein, PTPRT protein, PTCHD1 protein, MUC4 protein, CENPM protein, KLHL41 protein, FLG2 protein, GPR52 protein, MB21D1 protein, MT-ND1 protein, MT-CO2 protein, FAM21B protein or GSTM1 protein of any mammal species, including but not being limited to domestic and farm animals (cows, horses, pigs, sheep, goats, dogs, cats or rodents), primates and humans. Preferably, the POC1A protein, PTPRT protein, PTCHD1 protein, MUC4 protein, CENPM protein, KLHL41 protein, FLG2 protein, GPR52 protein, MB21D1 protein, MT-ND1 protein, MT-CO2 protein, FAM21B protein or GSTM1 protein is a human protein.

In order to measure the levels of the mRNA encoded by a given gene, the biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. In some embodiments, the RNA is extracted from formalin-fixed, paraffin embedded tissues. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinised and rehydrated. The sample is then lysed and RNA is extracted from the sample. Commercially available kits may be used for RNA extraction from paraffin samples, such as PureLink^{™} FFPE Total RNA Isolation Kit (Thermofisher Scientific Inc., US). Methods for RNA extraction from paraffin embedded tissues are disclosed, for example, in Rupp and Locker (1987) Lab Invest. 56:A67, and De Andres et al., BioTechniques 18:42044 (1995). Preferably, care is taken to avoid degradation of the RNA during the extraction process.

Various technologies are well-known in the art for deducing and/or measuring and/or detecting the levels of one or more transcripts in a cell. Such methods include hybridization-or sequence-based approaches. Hybridization-based approaches typically involve incubating fluorescently labelled cDNA with custom-made microarrays or commercial high-density oligo microarrays. Specialized microarrays have also been designed; for example, arrays with probes spanning exon junctions can be used to detect and quantify distinct spliced isoforms. Genomic tiling microarrays that represent the genome at high density have been constructed and allow the mapping of transcribed regions to a very high resolution, from several base pairs to -100 bp. Hybridization-based approaches are high throughput and relatively inexpensive, except for high-resolution tiling arrays that interrogate large genomes. However, these methods have several limitations, which include: reliance upon existing knowledge about genome sequence; high background levels owing to cross-hybridization; and a limited dynamic range of detection owing to both background and saturation of signals. Moreover, comparing expression levels across different experiments is often difficult and can require complicated normalization methods.

In contrast to microarray methods, sequence-based approaches directly determine the cDNA sequence. Initially, Sanger sequencing of cDNA or EST libraries was used, but this approach is relatively low throughput, expensive and generally not quantitative. Tag-based methods were developed to overcome these limitations, including serial analysis of gene expression (SAGE), cap analysis of gene expression (CAGE), and massively parallel signature sequencing (MPSS). These tag-based sequencing approaches are high throughput and can provide precise, digital gene expression levels. However, most are based on Sanger sequencing technology, and a significant portion of the short tags cannot be uniquely mapped to the reference genome. Moreover, only a portion of the transcript is analyzed, and isoforms are generally indistinguishable from each other. These disadvantages limit the use of traditional sequencing technology in measuring or detection mRNA levels.

The present methods can also involve a larger-scale analysis of mRNA levels, e.g., the detection of a plurality of biomarkers (e.g., 2-10, or 5-50, or 10-100, or 50-500 or more at one time). In addition, the methods described here can also involve the step of conducting a transcriptomic analysis (i.e., the analysis of the complete set of transcripts in a cell, and their quantity, for a specific developmental stage or physiological condition). Understanding the transcriptome can be important for interpreting the functional elements of the genome and revealing the molecular constituents of cells and tissues, and also for understanding development and disease and how the biomarkers disclosed herein are indicative or predictive of a particular condition (e.g., LM or LMS). The key aims of transcriptomics are: to catalogue all species of transcript, including mRNAs, non-coding RNAs and small RNAs; to determine the transcriptional structure of genes, in terms of their start sites, 5' and 3' ends, splicing patterns and other post-transcriptional modifications; and to quantify the changing expression levels of each transcript during development and under different conditions.

Recently, the development of novel high-throughput DNA sequencing methods has provided a new method for both mapping and quantifying transcriptomes. This method, termed RNAseq (RNA sequencing), has advantages over existing approaches for determining transcriptomes. Accordingly, in one embodiment, the expression level of the gene or genes used in the first method of the invention are determined by RNAseq.

As used herein "RNAseq" or "RNA-seq" refers to a transcriptomic approach where the total complement of RNAs from a given sample is isolated and sequenced using high-throughput next generation sequencing (NGS) technologies (e.g., SOLiD, 454, Illumina, or ION Torrent).

RNAseq uses deep-sequencing technologies. In general, a population of RNA (total or fractionated, such as poly(A)+) is converted to a library of cDNA fragments with adaptors attached to one or both ends. Each molecule, with or without amplification, is then sequenced in a high-throughput manner to obtain short sequences from one end (single-end sequencing) or both ends (pair-end sequencing). The reads are typically 30-400 bp, depending on the DNA-sequencing technology used. In principle, any high-throughput sequencing technology can be used for RNA-Seq, e.g., the Illumina IG18, Applied Biosystems SOLiD22 and Roche 454 Life Science systems have already been applied for this purpose. The Helicos Biosciences tSMS system is also appropriate and has the added advantage of avoiding amplification of target cDNA. Following sequencing, the resulting reads are either aligned to a reference genome or reference transcripts, or assembled de novo without the genomic sequence to produce a genome-scale transcription map that consists of both the transcriptional structure and/or level of expression for each gene.

Transcriptome analysis by next-generation sequencing (RNA-seq) allows investigation of a transcriptome at unsurpassed resolution. One major benefit is that RNA-seq is independent of a priori knowledge on the sequence under investigation.

The transcriptome can be profiled by high throughput techniques including SAGE, microarray, and sequencing of clones from cDNA libraries. For more than a decade, oligo nucleotide microarrays have been the method of choice providing high throughput and affordable costs. However, microarray technology suffers from well-known limitations including insufficient sensitivity for quantifying lower abundant transcripts, narrow dynamic range and biases arising from non-specific hybridizations. Additionally, microarrays are limited to only measuring known/annotated transcripts and often suffer from inaccurate annotations. Sequencing -based methods such as SAGE rely upon cloning and sequencing cDNA fragments. This approach allows quantification of mRNA abundance by counting the number of times cDNA fragments from a corresponding transcript are represented in a given sample, assuming that cDNA fragments sequenced contain sufficient information to identify a transcript. Sequencing-based approaches have a number of significant technical advantages over hybridization-based microarray methods. The output from sequence-based protocols is digital, rather than analog, obviating the need for complex algorithms for data normalization and summarization while allowing for more precise quantification and greater ease of comparison between results obtained from different samples. Consequently, the dynamic range is essentially infinite, if one accumulates enough sequence tags. Sequence-based approaches do not require prior knowledge of the transcriptome and are therefore useful for discovery and annotation of novel transcripts as well as for analysis of poorly annotated genomes. However, until recently the application of sequencing technology in transcriptome profiling has been limited by high cost, by the need to amplify DNA through bacterial cloning, and by the traditional Sanger approach of sequencing by chain termination.

The next-generation sequencing (NGS) technology eliminates some of these barriers, enabling massive parallel sequencing at a high but reasonable cost for small studies. The technology essentially reduces the transcriptome to a series of randomly fragmented segments of a few hundred nucleotides in length. These molecules are amplified by a process that retains spatial clustering of the PCR products, and individual clusters are sequenced in parallel by one of several technologies. Current NGS platforms include the Roche 454 Genome Sequencer, Illumina's Genome Analyzer, and Applied Biosystems' SOLiD. These platforms can analyze tens to hundreds of millions of DNA fragments simultaneously, generate giga-bases of sequence information from a single run, and have revolutionized SAGE and cDNA sequencing technology. For example, the 3' tag Digital Gene Expression (DGE) uses oligo-dT priming for first strand cDNA synthesis, generates libraries that are enriched in the 3' untranslated regions of polyadenylated mRNAs, and produces base cDNA tags.

In various embodiments the use of such sequencing technologies does not require the preparation of sequencing libraries. However, in certain embodiments the sequencing methods contemplated herein requires the preparation of sequencing libraries.

Any method for making high-throughput sequencing libraries can be used. An example of sequencing library preparation is described in U.S. Patent Application Publication No. US 2013/0203606, which is incorporated by reference in its entirety. In some embodiments, this preparation may take the coagulated portion of the sample from the droplet actuator as an assay input. The library preparation process is a ligation-based process, which includes four main operations: (a) blunt-ending, (b) phosphorylating, (c) A-tailing, and (d) ligating adaptors. DNA fragments in a droplet are provided to process the sequencing library. In the blunt-ending operation (a), nucleic acid fragments with 5'- and/or 3 '-overhangs are blunt-ended using T4 DNA polymerase that has both a 3 '-5' exonuclease activity and a 5'-3' polymerase activity, removing overhangs and yielding complementary bases at both ends on DNA fragments. In some embodiments, the T4 DNA polymerase may be provided as a droplet. In the phosphorylation operation (b), T4 polynucleotide kinase may be used to attach a phosphate to the 5'-hydroxyl terminus of the blunt-ended nucleic acid. In some embodiments, the T4 polynucleotide kinase may be provided as a droplet. In the A-tailing operation (c), the 3' hydroxyl end of a dATP is attached to the phosphate on the 5 '-hydroxyl terminus of a blunt-ended fragment catalyzed by exo-Klenow polymerase. In the ligating operation (d), sequencing adaptors are ligated to the A-tail. T4 DNA ligase is used to catalyze the formation of a phosphate bond between the A-tail and the adaptor sequence. In some embodiments involving cfDNA, end-repairing (including blunt-ending and phosphorylation) may be skipped because the cfDNA are naturally fragmented, but the overall process upstream and downstream of end repair is otherwise comparable to processes involving longer strands of DNA.

In another example, sequencing library preparation can involve the production of a random collection of adapter-modified DNA fragments (e.g., polynucleotides) that are ready to be sequenced. Sequencing libraries of polynucleotides can be prepared from DNA or RNA, including equivalents, analogs of either DNA or cDNA, for example, DNA or cDNA that is complementary or copy DNA produced from an RNA template, by the action of reverse transcriptase. The polynucleotides may originate in double-stranded form (e.g., dsDNA such as genomic DNA fragments, cDNA, PCR amplification products, and the like) or, in certain embodiments, the polynucleotides may originated in single-stranded form (e.g., ssDNA, RNA, etc.) and have been converted to dsDNA form.

By way of illustration, in certain embodiments, single stranded mRNA molecules may be copied into double-stranded cDNAs suitable for use in preparing a sequencing library. The precise sequence of the primary polynucleotide molecules is generally not material to the method of library preparation, and may be known or unknown. In one embodiment, the polynucleotide molecules are DNA molecules. More particularly, in certain embodiments, the polynucleotide molecules represent the entire genetic complement of an organism or substantially the entire genetic complement of an organism, and are genomic DNA molecules (e.g., cellular DNA, cell free DNA (cfDNA), etc.), that typically include both intron sequence and exon sequence (coding sequence), as well as non-coding regulatory sequences such as promoter and enhancer sequences. In certain embodiments, the primary polynucleotide molecules comprise human genomic DNA molecules, e.g., cfDNA molecules present in peripheral blood of a subject.

Preparation of sequencing libraries for some NGS sequencing platforms is facilitated by the use of polynucleotides comprising a specific range of fragment sizes. Preparation of such libraries typically involves the fragmentation of large polynucleotides (e.g. cellular genomic DNA) to obtain polynucleotides in the desired size range.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained from fresh tumor tissue or from OCT embedded frozen tissue. In another preferred embodiment samples can be obtained by laparoscopic myomectomy and then paraffin-embedded.

In order to normalize the values of mRNA expression among the different samples, it is possible to compare the expression levels of the mRNA of interest in the test samples with the expression of a control RNA. A "control RNA" as used herein, relates to RNA whose expression levels do not change or change only in limited amounts in tumor cells with respect to non-tumorigenic cells. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, IPO8, HPRT, GAPDH, PSMB4, tubulin and β-actin.

In one embodiment, the relative gene expression quantification is calculated according to the comparative threshold cycle (Ct) method using GAPDH, IPO8, HPRT, β-actin or PSMB4 as an endogenous control and commercial RNA controls as calibrators. Final results are determined according to the formula 2^{-(ΔCt sample-ΔCt calibrator)}, where ΔCT values of the calibrator and sample are determined by subtracting the Ct value of the target gene from the value of the control gene.

Suitable methods to determine gene expression levels at the mRNA level include, without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence in situ hybridization (FISH), including variants such as Flow-FISH, qFiSH and double fusion FISH (D-FISH), and the like.

In some embodiments, the determination of the expression levels of the gene or genes is carried out by exome-wide gene expression from RNAseq.

In some embodiments, the first method of the invention is based on a proteomic analysis.

The term "proteomic analysis" is used to refer to the analysis of the expression level of one or more proteins in a biological sample. Such analysis can, for example, be accomplished using mass spectrometry, two-dimensional gel electrophoresis, immunoassays, or by any other means for quantifying the level of protein expression in a sample.

In some embodiments, the determination of the expression levels of the gene or genes is carried out by measuring the expression levels of the protein or proteins or any functionally equivalent variant thereof encoded by said gene or genes.

In some embodiments, the first method of the invention comprises the determination of the expression levels of the protein encoded by the POC1A gene, PTPRT gene, PTCHD1 gene, MUC4 gene, CENPM gene, KLHL41 gene, FLG2 gene, GPR52 gene, MB21 D1 gene, MT-ND1 gene, MT-CO2 gene, FAM21B gene or GSTM1 gene, i.e. the POC1A protein, PTPRT protein, PTCHD1 protein, MUC4 protein, CENPM protein, KLHL41 protein, FLG2 protein, GPR52 protein, MB21D1 protein, MT-ND1 protein, MT-CO2 protein, FAM21B protein or GSTM1 protein or of variants thereof.

In some embodiments, the biological sample is a sample containing myometrial cells or RNA derived from myometrial cells. In yet another embodiment, the sample containing myometrial cells or RNA derived from myometrial cells is a myometrial biopsy.

In yet another embodiment, the sample containing myometrial cells is a biofluid. In a particular embodiment, the biofluid is selected from the group consisting of blood, plasma and serum.

In the second step, the first method of the invention comprises comparing said level of expression with a reference value.

The term "reference value", as used herein, refers to a laboratory value used as a reference for values/data obtained by laboratory examinations of subjects or samples collected from subjects. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time or from a non-cancerous tissue, or a value obtained from a sample from a different subject to the subject being tested. The reference value can be based on a large number of samples, such as from population of subjects of the chronological age matched group coinciding with that of the subject, or based on a pool of samples including or excluding the sample to be tested. Various considerations are taken into account when determining the reference value of the biomarker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group. In another embodiment, the quantity of the biomarker in a sample from a tested subject may be determined directly relative to the reference value (e.g., in terms of increase or decrease, or fold-increase or fold-decrease). Advantageously, this may allow to compare the quantity of the biomarker in the sample from the subject with the reference value (in other words to measure the relative quantity of any one or more biomarkers in the sample from the subject vis-a-vis the reference value) without the need to first determine the respective absolute quantities of said biomarker.

As used herein, the term "biomarker" refers to the expression level of a gene, a gene product, or of the protein encoded by said gene, as defined above.

Typically, reference values are the expression level of the gene being compared in a reference sample. The term "reference sample", as used herein, means at least a sample obtained from at least a subject (e.g., from an individual subject or from a pool of subjects) affected by uterine leiomyoma. Thus, in an embodiment, the reference value is the mean level of expression of the same gene or genes determined in a group of samples from a group of subjects with uterine leiomyoma. It will be understood that the "reference sample" defined as "a group of samples from a group of subjects" means a pool of samples obtained from a pool of subjects with uterine leiomyoma. In an embodiment, the pool of samples from leiomyoma patients are obtained from a myometrial biopsy from subjects affected by uterine leiomyoma. In a preferred embodiment, the pool of samples from leiomyoma patients are obtained from a biofluid sample from subjects affected by uterine leiomyoma.

The expression profile of the genes in the reference sample can preferably, be generated from a population of two or more individuals. The population, for example, can comprise 3, 4, 5, 10, 15, 20, 30, 40, 50 or more individuals with uterine leiomyoma. Furthermore, the expression profile of the gene or genes in the reference sample and in the sample of the individual that is going to be diagnosed according to the methods of the present invention can be generated from one individual, provided that said individual is affected by uterine leiomyoma.

Once this reference value is established, the level of the biomarker expressed in the tumor tissue or biofluid from subjects can be compared with the reference value, and thus be assigned a level of deviation with respect to a reference value. The "deviation" can be either an increase or a decrease in the expression level of the biomarker with respect to the reference value. For example, an increase in the expression level of a gene above the reference value of at least 1.1-fold, 1.5-fold, 2-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level, or an upregulation in the expression level of a gene in a sample with respect to the reference value. Similarly, the expression level of a gene is considered increased in a sample of the subject under study when its expression levels increase with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. On the other hand, a decrease in the expression level of a gene below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level, or a downregulation in the expression level of a gene in a sample with respect to the reference value. Similarly, the expression level of a gene is considered decreased in a sample of the subject under study when its expression levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent). The comparison of the expression levels of the gene or genes of interest with the reference value allows distinguishing between uterine leiomyosarcoma or uterine leiomyoma.

In some embodiments, the patient is detected as having leiomyosarcoma if the expression level of the gene or genes under examination is/are increased with respect to the expression level of said gene or genes found in leiomyoma samples, which are the POC1A gene, the PTPRT gene, the MUC4 gene, the FLG2 gene, the GPR52 gene, the MT-ND1 gene or the MT-CO2 gene having a log2FoldChange higher than 0 (i.e., if the log2FoldChange value is a positive value).

In some embodiments, the patient is detected as having leiomyosarcoma if the expression level of the gene or genes under examination is/are decreased with respect to the expression level of said gene or genes found in leiomyoma samples, which is are the PTCHD1 gene, the CENPM gene, the KLHL41 gene, the MB21D1 gene, the FAM21B gene or the GSTM1 gene having a log2FoldChange lower than 0 (i.e., if the log2FoldChange value is a negative value).

In a preferred embodiment, the deviation of the expression level with respect to the reference value in the POC1A gene, the PTPRT gene, the MUC4 gene, the FLG2 gene, the GPR52 gene, the MT-ND1 gene or the MT-CO2 gene is an increase and wherein the deviation of the expression level with respect to the reference value in the PTCHD1 gene, the CENPM gene, the KLHL41 gene, the MB21D1 gene, the FAM21B gene or the GSTM1 gene is a decrease.

In another embodiment, the first method according to the invention comprises measuring the expression level of the POC1A, PTPRT, PTCHD1, MUC4, CENPM, KLHL41, FLG2, GPR52, MB21D1, MT-ND1, MT-CO2, FAM21B and GSTM1 genes.

In some embodiments, the first method according to the invention allows the diagnosis of uterine leiomyosarcoma when the deviation in the level of expression of the gene or genes is/are of at least two fold with respect to the reference value or values for said gene or genes, said reference value being the expression level of the same gene or genes determined in at least a sample from at least a subject with uterine leiomyoma.

In some embodiments, the first method according to the invention is carried out in a patient that has been previously identified as having a uterine myometrial tumor, being either uterine leiomyosarcoma or uterine leiomyoma by imaging examination, preferably by ultrasonography.

### Second method of the invention (method based on transcriptomic analysis)

The authors of the present invention have carried out a comparative transcriptomic analysis between histologically confirmed LM (n = 72) and LMS (n = 23) tumors. The results have revealed 440 genes that were differentially expressed obtained from 5 selected comparisons between LMS and LM liquid biopsy samples. Selected group comparisons differed in demographic and technical characteristics, such as the age of the subject, or the freezing time of the sample prior to the RNA extraction and data quality. This difference in the transcriptomic profiles allows distinguishing between one disease or the other in a subject by analyzing the RNA composition in a sample from the subject and classifying the subject affected by LMS or LM using a transcriptomic analysis-based method. Thus, in a second aspect, the invention relates to an *in vitro* method for distinguishing between uterine leiomyosarcoma or uterine leiomyoma in a subject suspected of having one of these conditions (hereinafter referred to as "the second method of the invention"), the method comprising:
(i) measuring the level of expression of at least one gene selected from the list shown in Table 2, the list shown in Table 3, the list shown in Table 4, the list shown in Table 6 or the list shown in Table 7 in a biological sample obtained from the subject, and
(ii) comparing said level of expression with a reference value, and
wherein a deviation in the level of expression of said at least one gene with respect to said reference value is indicative that the subject is affected by from uterine leiomyosarcoma.

The terms and expressions "distinguishing between", "uterine leiomyoma", "uterine leiomyosarcoma", "expression level", "biological sample" and "subject" have been defined in the context of the first method of the invention and apply equally to the second method of the invention.

In a first step, the second method of the invention comprises the determination of the level of expression of at least one gene selected from the list shown in Table 2, the list shown in Table 3, the list shown in Table 4, the list shown in Table 6 or the list shown in Table 7 in a biological sample obtained from the subject whose diagnosis is to be determined.

In some embodiments, the first step of the second method of the invention comprises the determination of the expression levels of at least 3, at least 15, at least 10, at least 15, at least 20, at least 25, at least 30, at least 35 or the 37 genes listed in Table 2.

In some embodiments, the first step of the second method of the invention comprises the determination of the expression levels of at least 3, at least 5, at least 10, at least 15, at least 20, at least 25, at least 30, at least 35, at least 40, at least 45, at least 50, or the 55 genes listed in Table 3.

In some embodiments, the first step of the second method of the invention comprises the determination of the expression levels of at least 5, at least 10, at least 15, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, at least 200, at least 210, at least 220, at least 230, at least 240, at least 250, at least 260, at least 270, at least 280, at least 290, at least 300, at least 310, at least 320, at least 330, at least 340, at least 350, at least 360, at least 370 or the 378 genes listed in Table 4.

In some embodiments, the first step of the second method of the invention comprises the determination of the expression levels of at least 3, at least 5, at least 10 or the 13 genes listed in Table 6.

In some embodiments, the first step of the second method of the invention comprises the determination of the expression levels of at least 5, at least 10, at least 15, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, at least 200, at least 210, at least 220, at least 230 or the 234 genes listed in Table 7.

In a particular embodiment, the sample from the subject according to the methods of the present invention is selected from the group consisting of blood, plasma and serum, and a tissue sample; more preferably from the group consisting of plasma and a tissue sample.

In some embodiments, the sample wherein the expression level of the genes comprised in Table 2, Table 3, Table 4, Table 6 or Table 7 is determined can be any sample containing cells from the potential tumor. In a particular embodiment, the sample containing cells from the potential tumor is a potential tumor tissue or a portion thereof. In a more particular embodiment, said potential tumor tissue sample is a uterine tissue sample from a patient in which the method for distinguishing between uterine leiomyosarcoma or uterine leiomyoma in a subject suspected of having uterine leiomyosarcoma or uterine leiomyoma is to be carried out. Said sample can be obtained by conventional methods, e.g., biopsy, surgical excision, or aspiration, by using methods well known to those of ordinary skill in the related medical arts. Methods for obtaining the sample from the biopsy have been explained in the context of the first method according to the invention and apply equally to the second method of the invention. In some embodiments, the sample has been obtained by hysterectomy or laparoscopic/laparotomic myomectomy.

In a particular embodiment of the second method of the invention, the sample wherein the expression levels of the genes comprised in Table 2, Table 3, Table 4, Table 6 or Table 7 are determined, is a tumor sample obtained by hysterectomy or laparoscopic/laparotomic myomectomy.

In another particular embodiment, the sample containing the cells from the potential tumor is a biofluid. In a more particular embodiment, said biofluid is selected from the group consisting of blood, plasma and serum from a patient in which the method for distinguishing between uterine leiomyosarcoma or uterine leiomyoma in a subject suspected of having uterine leiomyosarcoma or uterine leiomyoma is to be carried out. Said biofluid sample can be obtained by conventional methods well known to those of ordinary skill in the related arts.

In yet another particular embodiment of the second method of the invention, the sample wherein the expression levels of the genes comprised in Table 2, Table 3, Table 4, Table 6 or Table 7 are determined, is a biofluid obtained by any art-appropriate means.

Gene expression levels can be quantified by measuring the messenger RNA (mRNA) levels of the gene or of the protein encoded by said gene, i.e. the protein or proteins encoded by the gene or genes comprised in Table 2, Table 3, Table 4, Table 6 or Table 7 or of variants thereof. The protein variants of the protein or proteins encoded by the gene or genes comprised in Table 2, Table 3, Table 4, Table 6 or Table 7 include all the physiologically relevant post-translational chemical modifications forms of the protein, for example, glycosylation, phosphorylation, acetylation, etc., provided that the functionality of the protein is maintained. Said term encompasses the protein or proteins encoded by the gene or genes comprised in Table 2, Table 3, Table 4, Table 6 or Table 7 of any mammal species, including but not being limited to domestic and farm animals (cows, horses, pigs, sheep, goats, dogs, cats or rodents), primates and humans. Preferably, said protein or proteins encoded by the gene or genes comprised in Table 2, Table 3, Table 4, Table 6 or Table 7 is a human protein.

The measurement of the levels of the mRNA encoded by a given gene has been defined in the context of the first method according to the invention and apply equally to the second method of the invention. Accordingly, in one embodiment, the expression level of the gene or genes used in the second method of the invention are determined by RNAseq.

The term "RNAseq", the transcriptome analysis by next-generation sequencing, next-generation sequencing, sequencing libraries, the determination of the expression level using mRNA, the normalization of the mRNA expression values, the relative gene expression quantification and suitable methods to determine gene expression levels at the mRNA level have been defined in the first method according to the invention and apply equally to the second method of the invention.

In some embodiments, the determination of the expression levels of the gene or genes used in the second method of the invention is carried out by exome-wide gene expression from RNAseq.

In some embodiments, the second method of the invention is based on a proteomic analysis.

The term "proteomic analysis" has been defined in the first method according to the invention and apply equally to the second method of the invention.

In some embodiments, the determination of the expression levels of the gene or genes is carried out by measuring the expression levels of the protein or proteins or any functionally equivalent variant thereof encoded by said gene or genes.

In some embodiments, the second method of the invention comprises the determination of the expression levels of the protein or proteins encoded by the gene or genes comprised in Table 2, Table 3, Table 4, Table 6 or Table 7, i.e. the protein or proteins encoded by the gene or genes comprised in Table 2, Table 3, Table 4, Table 6 or Table 7 or of variants thereof.

In some embodiments, the biological sample is a sample containing myometrial cells or RNA derived from myometrial cells. In yet another embodiment, the sample containing myometrial cells or RNA derived from myometrial cells is a myometrial biopsy.

In yet another embodiment, the sample containing myometrial cells is a biofluid. In a particular embodiment, the biofluid is selected from the group consisting of blood, plasma and serum.

In the second step, the second method of the invention comprises comparing said level of expression with a reference value. The term "reference value" has been defined in the first method according to the invention and apply equally to the second method of the invention.

Thus, in another embodiment of the second method of the invention, the reference value is the mean level of expression of the same gene or genes determined in a group of samples from a group of subjects with uterine leiomyoma. The expression "a group of samples from a group of subjects" has been defined in the first method according to the invention and apply equally to the second method of the invention.

In an embodiment, the pool of samples from leiomyoma patients are obtained from a myometrial biopsy from subjects affected by uterine leiomyoma. In a preferred embodiment, the pool of samples from leiomyoma patients are obtained from a biofluid sample from subjects affected by uterine leiomyoma.

The expression profile of the genes in the reference sample can preferably, be generated from a population of two or more individuals. The population, for example, can comprise 3, 4, 5, 10, 15, 20, 30, 40, 50 or more individuals with uterine leiomyoma. Furthermore, the expression profile of the gene or genes in the reference sample and in the sample of the individual that is going to be diagnosed according to the methods of the present invention can be generated from one individual, provided that said individual is affected by uterine leiomyoma.

Once this reference value is established, the level of the biomarker expressed in the tumor tissue or biofluid from subjects can be compared with the reference value, and thus be assigned a level of deviation with respect to a reference value.

The terms "reference sample", "biomarker" and "deviation" have been defined in the first method according to the invention and apply equally to the second method of the invention.

In some embodiments, the patient is detected as having leiomyosarcoma if the expression level of the gene or genes under examination is/are increased with respect to the expression level of said gene or genes found in leiomyoma samples, which are defined in Table 2, in Table 3, in Table 4, in Table 6 or in Table 7 as genes having a log2FoldChange higher than 0 (i.e., if the log2FoldChange value is a positive value).

In some embodiments, the patient is detected as having leiomyosarcoma if the expression level of the gene or genes under examination is/are decreased with respect to the expression level of said gene or genes found in leiomyoma samples, which is are defined in Table 2, in Table 3, in Table 4, in Table 6 or in Table 7 as genes having a log2FoldChange lower than 0 (i.e., if the log2FoldChange value is a negative value).

In a preferred embodiment, the deviation in the level of expression of the gene or genes is a downregulation in the sample with respect to the reference value if the log2FoldChange value for said at least one gene as indicated in the Table is a negative value, or the deviation in the level of expression of the gene or genes is an upregulation in the sample with respect to the reference value if the log2FoldChange value for said at least one gene as indicated in the Table is a positive value.

In another embodiment, the second method according to the invention comprises measuring the expression level of all the genes comprised in Table 2, Table 3, Table 4, Table 6 or Table 7.

In some embodiments, the second method according to the invention allows the diagnosis of uterine leiomyosarcoma when the deviation in the level of expression of the gene or genes is/are of at least two fold with respect to the reference value or values for said gene or genes, said reference value being the expression level of the same gene or genes determined in at least a sample from at least a subject with uterine leiomyoma.

In some embodiments, the second method according to the invention is carried out in a patient that has been previously identified as having a uterine myometrial tumor, being either uterine leiomyosarcoma or uterine leiomyoma by imaging examination, preferably by ultrasonography.

In some embodiments, the biological sample of the second method of the invention, in which the determination of the levels of expression of the gene or genes is to be carried out, can be sorted in groups differing in specific characteristics, such as, but not limited to, groups with specific demographic characteristics or specific technical characteristics. Illustrative, non-limitative examples of specific demographic or technical characteristics include the age of the subject, or the freezing time of the sample prior to the RNA extraction and data quality.

Accordingly, in some embodiments according to the second method of the invention:
(i) if the biological sample has been frozen for less than 6 months, then step (i) of the second method of the invention comprises measuring the level of expression of at least one gene selected from the list shown in Table 4,
(ii) if the biological sample derives from a subject over 41 years of age, then step (i) of the second method of the invention comprises measuring the level of expression of at least one gene selected from the list shown in Table 6, or
(iii) if the biological sample has been frozen for less than 6 months and it derives from a subject over 41 years of age, then step (i) of the second method of the invention comprises measuring the level of expression of at least one gene selected from the list shown in Table 7.

### Methods for identifying a subject to receive a suitable therapy based on the first method of the invention or the second method of the invention

The invention also provides a method for identifying a subject suspected of having uterine leiomyosarcoma as a candidate to receive a suitable therapy to treat uterine leiomyosarcoma.

Thus, in a third aspect, the invention relates to an *in vitro* method for identifying a subject suspected of having uterine leiomyosarcoma as a candidate to receive a suitable therapy to treat uterine leiomyosarcoma (hereinafter referred to as "the third method of the invention"), the method comprising:
(i) determining whether the subject is affected by uterine leiomyosarcoma following any of the first method of the invention or the second method of the invention; and
(ii) designating said subject as a candidate to receive a suitable therapy to treat uterine leiomyosarcoma if the subject is diagnosed as having uterine leiomyosarcoma.

In a first step, the *in vitro* method for identifying a subject suspected of having uterine leiomyosarcoma as a candidate to receive a suitable therapy to treat uterine leiomyosarcoma comprises determining whether the subject is affected by uterine leiomyosarcoma by using any of the first method of the invention or the second method of the invention.

In a second step, the method comprises designating a subject as a candidate to be treated with a therapy suitable for the treatment of uterine leiomyosarcoma if the patient is diagnosed as having uterine leiomyosarcoma.

In some embodiments, when the subject is being diagnosed with uterine leiomyosarcoma, the subject is designated to be treated with a therapy selected from the group consisting of surgery, radiation therapy, chemotherapy, hormonal therapy and targeted therapy.

In some embodiments, the surgery is a simple hysterectomy, radical hysterectomy or bilateral salpingo-oophorectomy.

In some embodiments, the therapy is radiation therapy. In the present context, the terms "radiation therapy" or "radiotherapy" refer to the treatment of cancer cells with ionizing radiation in order to control or kill malignant cells. It is meant to include, for example, fractionated radiation therapy, non-fractionated radiation therapy and super-fractionated radiation therapy, as well as a combination of radiation and chemotherapy. The type of radiation may further include ionizing (y) radiation, particle radiation, low energy transfer (LET), high energy transfer (HET), X-ray radiation, UV radiation, infrared radiation, visible light, photosensitizing radiation, etc.

In some embodiments, the chemotherapy includes one or more drugs selected from the group consisting of dacarbazine (DTIC), docetaxel, doxorubicin, epirubicin, gemcitabine, ifosfamide, paclitaxel, temozolomide, trabectedin and vinorelbine.

In some embodiments, the therapy is an hormonal therapy. The term "hormonal therapy", as used herein, comprises:
- progestin,
- an agonist of the gonadotropin-releasing hormone such as goserelin or leuprolide, leuprorelin acetate, leuprorelin acetate sustained release depot (ATRIGEL), triptorelin pamoate, buserelin, naferelin, histrelin, goserelin, deslorelin, degarelix, ozarelix, ABT-620 (elagolix), TAK-385 (relugolix), EP-100, KLH-2109 or triptorelinand goserelin acetate, or
- an aromatase inhibitor, which is defined as a compound which inhibits estrogen production, for instance, the conversion of the substrates androstenedione and testosterone to estrone and estradiol, respectively, and that includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole

In some embodiments, the therapy is a targeted therapy. The term "targeted therapy", as used herein, refers to drugs which attack specific genetic mutations within cancer cells, such as leiomyosarcoma while minimizing harm to healthy cells. In some embodiments, the targeted therapy comprises the use of pazopanib.

### Therapeutic methods coupled to the first method of the invention or the second method of the invention

The invention also provides methods for the treatment of subjects which have been identified as having leiomyosarcomas by any of the first method of the invention or the second method of the invention, wherein if the subject has been diagnosed as having a leiomyosarcoma, the subject is treated with a therapy suitable for the treatment of leiomyosarcoma.

Thus, in another aspect, the invention relates to a method for treating uterine leiomyosarcoma in a subject in need thereof comprising the administration of a therapy suitable for the treatment of uterine leiomyosarcoma, wherein the subject to be treated has been identified using any of the first method of the invention or the second method of the invention.

In some embodiments, the therapy is selected from the group consisting of surgery, radiation therapy, chemotherapy, hormonal therapy and targeted therapy.

The term "treatment", as used herein, comprises any type of therapy, which aims at terminating, ameliorating, delaying and/or reducing the severity of a clinical condition as described herein. Thus, "treatment," "treating," and the like, as used herein, refer to obtaining a desired pharmacologic and/or physiologic effect, covering any treatment of a pathological condition or disorder in a mammal, including a human. The effect may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

The therapeutic method according to the invention is applied to patients which have been diagnosed as having leiomyosarcoma by using any of the first method of the invention or the second method of the invention. In some embodiments, the method comprises a first step in which any of the first method of the invention or the second method of the invention is applied to the subject, a second step in which subjects diagnosed as having leiomyosarcoma are identified, and a third step in which the subjects are treated with a therapy suitable for the treatment of leiomyosarcoma.

In some embodiments, when the subject is being diagnosed with uterine leiomyosarcoma, the subject is designated to be treated with a therapy selected from the group consisting of surgery, radiation therapy, chemotherapy, hormonal therapy and targeted therapy.

Suitable surgical therapies, radiation therapies, chemotherapies, hormonal therapies or targeted therapies have been described in the context of the third method of the invention and are equally applicable to the therapeutic methods according to the invention.

In another aspect, the invention relates to a chemotherapeutic agent, hormonal agent and/or targeted agent for use in the treatment of uterine leiomyosarcoma, wherein the subject to be treated has been identified by any of the first method of the invention or the second method of the invention.

The term "chemotherapeutic agent", as used herein, refers to standard chemotherapy drugs, which generally attack any quickly dividing cell, targeted therapy agents and immunomodulatory agents. Illustrative non-limitative examples of chemotherapeutic agents include dacarbazine (DTIC), docetaxel, doxorubicin, epirubicin, gemcitabine, ifosfamide, paclitaxel, temozolomide, trabectedin or vinorelbine.

The term "hormonal agent", as used herein, comprises:
- progestin,
- an agonist of the gonadotropin-releasing hormone such as goserelin or leuprolide, leuprorelin acetate, leuprorelin acetate sustained release depot (ATRIGEL), triptorelin pamoate, buserelin, naferelin, histrelin, goserelin, deslorelin, degarelix, ozarelix, ABT-620 (elagolix), TAK-385 (relugolix), EP-100, KLH-2109 or triptorelinand goserelin acetate, or
- an aromatase inhibitor, which is defined as a compound which inhibits estrogen production, for instance, the conversion of the substrates androstenedione and testosterone to estrone and estradiol, respectively, and that includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole

The term "targeted agent", as used herein, refers to drugs which attack specific genetic mutations within cancer cells, such as leiomyosarcoma while minimizing harm to healthy cells. In some embodiments, the targeted therapy comprises the use of pazopanib.

### Kit of the invention and uses thereof

In another aspect, the invention relates to a kit, package and/or device that comprises reagents adequate for implementing any of the first method of the invention, the second method of the invention or the third method of the invention. It will be understood that, depending on the nature of the method, the reagents adequate for its implementation will vary.

In the context of the present invention, "kit" is understood as a product containing the different reagents required for carrying out the methods of the invention packaged such that it allows being transported and stored. The materials suitable for the packaging of the components of the kit include glass, plastic (polyethylene, polypropylene, polycarbonate, and the like), bottles, vials, paper, sachets, and the like. Where there are more than one component in a kit they may be packaged together if suitable or the kit will generally contain a second, third or other additional container into which the additional components may be separately placed. However, in some embodiments, certain combinations of components may be packaged together comprised in one container means. A kit can also include a means for containing any reagent containers in close confinement for commercial sale. Such containers may include injection or blow-molded plastic containers into which the desired vials are retained. One or more compositions of a kit can be lyophilized. In some embodiments, all compositions of a kit of the disclosure will be lyophilized. In some embodiments, a kit of the disclosure with one or more lyophilized agents will be supplied with a re-constitution buffer. Reagents and components of kits may be comprised in one or more suitable container means. A container means may generally comprise at least one vial, test tube, flask, bottle, syringe or other container means, into which a component may be placed, and preferably, suitably aliquoted.

Furthermore, kits according to the invention can also comprise one or more reagents for preparing crude cell lysates and/or reagents for extracting, isolating and/or purification of nucleic acids from a sample. Additional components can comprise particles with affinity for nucleic acids and/or solid supports with affinity for nucleic acids, one or more wash buffers, binding enhancers, binding solutions, polar solvents, alcohols, elution buffers, filter membranes and/or columns for isolation of DNA/RNA. A kit may further comprise reagents for downstream processing of an isolated nucleic acid and may include without limitation at least one RNase inhibitor; at least one cDNA construction reagents (such as reverse transcriptase); one or more reagents for amplification of RNA, one or more reagents for amplification of DNA including primers, reagents for purification of DNA, probes for detection of specific nucleic acids. Furthermore, the kits of the invention can contain instructions for the simultaneous, sequential, or separate use of the different components that are in the kit. Said instructions can be in the form of printed material or in the form of an electronic medium capable of storing instructions such that they can be read by a subject, such as electronic storage media (magnetic disks, tapes, and the like), optical media (CD-ROM, DVD), and the like. The media may additionally or alternatively contain Internet addresses providing said instructions.

In some embodiments, the kit comprises primers or probes adequate for the detection of the expression levels of one or more of the genes, the expression levels of which are determined in the first method of the invention or in the second method of the invention.

In some embodiments, when any of the first method of the invention or the second method of the invention is based on the determination of the expression levels of one or more genes, the kits comprise primers or probes adequate for the detection of the expression levels of said one or more genes.

The term "primer" as used herein refers to oligonucleotides that can specifically hybridize to a target polynucleotide sequence, due to the sequence complementarity of at least part of the primer within a sequence of the target polynucleotide sequence. A primer can have a length of at least 8 nucleotides, typically 8 to 70 nucleotides, usually of 18 to 26 nucleotides. For proper hybridization to the target sequence, a primer can have at least 75 percent, at least 80 percent, at least 85 percent, at least 90 percent, or at least 95 percent sequence complementarity to the hybridized portion of the target polynucleotide sequence. Oligonucleotides useful as primers may be chemically synthesized according to the solid phase phosphoramidite triester method first described by Beaucage and Caruthers, Tetrahedron Letts. (1981) 22: 1859-1862, using an automated synthesizer, as described in Needham-Van Devanter et al, Nucleic Acids Res. (1984) 12: 6159-6168. Primers are useful in nucleic acid amplification reactions in which the primer is extended to produce a new strand of the polynucleotide. Primers can be readily designed by a skilled artisan using common knowledge known in the art, such that they can specifically anneal to the nucleotide sequence of the target nucleotide sequence of the at least one biomarker provided herein. Usually, the 3' nucleotide of the primer is designed to be complementary to the target sequence at the corresponding nucleotide position, to provide optimal primer extension by a polymerase.

The term "probe" as used herein refers to oligonucleotides or analogs thereof that can specifically hybridize to a target polynucleotide sequence, due to the sequence complementarity of at least part of the probe within a sequence of the target polynucleotide sequence. Exemplary probes can be, for example DNA probes, RNA probes, or protein nucleic acid (PNA) probes. A probe can have a length of at least 8 nucleotides, typically 8 to 70 nucleotides, usually of 18 to 26 nucleotides. For proper hybridization to the target sequence, a probe can have at least 75 percent, at least 80 percent, at least 85 percent, at least 90 percent, or at least 95 percent sequence complementarity to hybridized portion of the target polynucleotide sequence. Probes can also be chemically synthesized according to the solid phase phosphoramidite triester method as described above. Methods for preparation of DNA and RNA probes, and the conditions for hybridization thereof to target nucleotide sequences, are described in Molecular Cloning: A Laboratory Manual, J. Sambrook et al., eds., 2nd edition. Cold Spring Harbor Laboratory Press, 1989, Chapters 10 and 11.

In a preferred embodiment, the reagents adequate for the determination of the expression levels of one or more genes comprise at least 10%, at least 20%, at least 30%, at least 40%, at least 50%, at least 60%, at least 70%, at least 80%, at least 90% or at least 100% of the total amount of reagents adequate for the determination of the expression levels of genes forming the kit.

In another embodiment, the invention relates to the use of the kit of the invention for distinguishing between uterine leiomyosarcoma or uterine leiomyoma in a subject suspected of having one of these conditions, or for identifying a subject as a candidate to receive a suitable therapy to treat uterine leiomyosarcoma.

### Computer Systems and Devices suitable for carrying out the methods of the invention

The methods of the invention can be performed using software, hardware, firmware, hardwiring, or combinations of any of these.

Accordingly, in another aspect, the invention relates to a computer-implemented method, wherein the method is any of the first method of the invention, the second method of the invention or the third method of the invention. In another aspect, the invention relates to a computer comprising instructions for carrying out any of the first method of the invention, the second method of the invention or the third method of the invention.
The invention is defined below by means of the following aspects:
1. An *in vitro* method for distinguishing between uterine leiomyosarcoma or uterine leiomyoma in a subject suspected of having one of these conditions, the method comprising:
   (i) measuring the level of expression of at least one gene selected from POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene in a biological sample obtained from the subject, and
   (ii) comparing said level of expression with a reference value,
   wherein a deviation in the level of expression of said at least one gene with respect to said reference value is indicative that the subject is affected by uterine leiomyosarcoma.
2. The method according to aspect 1, wherein the deviation of the expression level with respect to the reference value in the POC1A gene, the PTPRT gene, the MUC4 gene, the FLG2 gene, the GPR52 gene, the MT-ND1 gene or the MT-CO2 gene is an increase and/or wherein the deviation of the expression level with respect to the reference value in the PTCHD1 gene, the CENPM gene, the KLHL41 gene, the MB21D1 gene, the FAM21B gene or the GSTM1 gene is a decrease.
3. An *in vitro* method for distinguishing between uterine leiomyosarcoma or uterine leiomyoma in a subject suspected of having one of these conditions, the method comprising:
   (i) measuring the level of expression of at least one gene selected from the list shown in Table 2, the list shown in Table 3, the list shown in Table 4, the list shown in Table 6 or the list shown in Table 7 in a biological sample obtained from the subject, and
   (ii) comparing said level of expression with a reference value,
   wherein a deviation in the level of expression of said at least one gene with respect to said reference value is indicative that the subject is affected by uterine leiomyosarcoma.
4. The method according to aspect 3, wherein the deviation in the level of expression of the at least one gene is a downregulation in the sample with respect to the reference value if the log2FoldChange value for said at least one gene as indicated in the Table is a negative value, or wherein the deviation in the level of expression of the at least one gene is an upregulation in the sample with respect to the reference value if the log2FoldChange value for said at least one gene as indicated in the Table is a positive value.
5. The method according to aspect 1 or 3, wherein the reference value is the mean level of expression of the same gene or genes determined in a group of samples from a group of subjects with uterine leiomyoma.
6. The method according to aspect 1, wherein the method comprises measuring the expression level of the POC1A, PTPRT, PTCHD1, MUC4, CENPM, KLHL41, FLG2, GPR52, MB21D1, MT-ND1, MT-CO2, FAM21B and GSTM1 genes.
7. The method according to aspect 3, wherein the method comprises measuring the expression level of all the genes comprised in Table 2, Table 3, Table 4, Table 6 or Table 7.
8. The method according to aspect 3, wherein:
   (i) if the biological sample has been frozen for less than 6 months, then step (i) comprises measuring the level of expression of at least one gene selected from the list shown in Table 4,
   (ii) if the biological sample derives from a subject over 41 years of age, then step (i) comprises measuring the level of expression of at least one gene selected from the list shown in Table 6, or
   (iii) if the biological sample has been frozen for less than 6 months and it derives from a subject over 41 years of age, then step (i) comprises measuring the level of expression of at least one gene selected from the list shown in Table 7.
9. The method according to any one of the preceding aspects, the method comprising diagnosing uterine leiomyosarcoma when the deviation in the level of expression of the at least one gene is of at least two fold with respect to the reference value, said reference value being the expression level of the same gene or genes determined in at least a sample from at least a subject with uterine leiomyoma.
10. The method according to any one of the preceding aspects, wherein the subject has been previously identified as having a myometrial tumor by imaging examination, preferably by ultrasonography.
11. The method according to any one of the preceding aspects, wherein the determination of the expression levels of one or more genes is carried out by exome-wide gene expression from RNAseq.
12. The method according to any one of the preceding aspects, wherein the biological sample is a sample containing myometrial cells or RNA derived from myometrial cells.
13. The method according to aspect 12, wherein the sample containing myometrial cells or RNA derived from myometrial cells is a myometrial biopsy or a biofluid.
14. The method according to aspect 13, wherein the biofluid is selected from the group consisting of blood, plasma and serum.
15. An *in vitro* method for identifying a subject suspected of having uterine leiomyosarcoma as a candidate to receive a suitable therapy to treat uterine leiomyosarcoma, the method comprising:
   (i) determining whether the subject is affected by uterine leiomyosarcoma following the method of any one of aspects 1 to 14; and
   (ii) designating said subject as a candidate to receive a suitable therapy to treat uterine leiomyosarcoma if the subject is diagnosed as having uterine leiomyosarcoma.
16. The method according to aspect 15, wherein the therapy suitable for the treatment of uterine leiomyosarcoma is selected from the group consisting of surgery, radiation therapy, chemotherapy, hormonal therapy and targeted therapy.
17. The method according to aspect 16, wherein the surgery is simple hysterectomy, radical hysterectomy or bilateral salpingo-oophorectomy, wherein radiation therapy includes ionizing (y) radiation, particle radiation, low energy transfer (LET), high energy transfer (HET), X-ray radiation, UV radiation, infrared radiation, visible light, photosensitizing radiation, wherein the chemotherapy includes one or more drugs selected from the group consisting of dacarbazine (DTIC), docetaxel, doxorubicin, epirubicin, gemcitabine, ifosfamide, paclitaxel, temozolomide, trabectedin and vinorelbine, wherein the hormonal therapy comprises progestin, an agonist of the gonadotropin-releasing hormone or an aromatase inhibitor, and/or wherein the targeted therapy includes pazopanib.
18. A method for treating uterine leiomyosarcoma in a subject in need thereof comprising the administration of a therapy suitable for the treatment of uterine leiomyosarcoma, wherein the subject to be treated has been identified using a method according to any of aspects 1 to 14.
19. The method according to aspect 18, wherein the therapy suitable for the treatment of uterine leiomyosarcoma is selected from the group consisting of surgery, radiation therapy, chemotherapy, hormonal therapy and targeted therapy.
20. The method according to aspect 19, wherein the surgery is simple hysterectomy, radical hysterectomy or bilateral salpingo-oophorectomy, wherein radiation therapy includes ionizing (y) radiation, particle radiation, low energy transfer (LET), high energy transfer (HET), X-ray radiation, UV radiation, infrared radiation, visible light, photosensitizing radiation, wherein the chemotherapy includes one or more drugs selected from the group consisting of dacarbazine (DTIC), docetaxel, doxorubicin, epirubicin, gemcitabine, ifosfamide, paclitaxel, temozolomide, trabectedin and vinorelbine, wherein the hormonal therapy comprises progestin, an agonist of the gonadotropin-releasing hormone or an aromatase inhibitor, and/or wherein the targeted therapy includes pazopanib.
21. A chemotherapeutic agent, hormonal agent and/or targeted agent for use in the treatment of uterine leiomyosarcoma, wherein the subject to be treated has been identified by a method according to any of aspects 1 to 14.
22. The chemotherapeutic agent, hormonal agent and/or targeted agent according to aspect 21, wherein the chemotherapeutic agent includes one or more drugs selected from the group consisting of dacarbazine (DTIC), docetaxel, doxorubicin, epirubicin, gemcitabine, ifosfamide, paclitaxel, temozolomide, trabectedin and vinorelbine, wherein the hormonal agent comprises progestin, an agonist of the gonadotropin-releasing hormone or an aromatase inhibitor, and/or wherein the targeted agent includes pazopanib.
23. A kit, package and/or device comprising reagents adequate for implementing the methods according to any one of aspects 1 to 20.
24. The kit, package and/or device according to aspect 23, wherein the reagents comprise primers or probes adequate for the detection of the expression levels of one or more of the genes, the expression levels of which are determined in the methods according to any of aspects 1 to 20.
25. Use of a kit according to aspect 23 or 24 for distinguishing between uterine leiomyosarcoma or uterine leiomyoma in a subject suspected of having one of these conditions, or for identifying a subject as a candidate to receive a suitable therapy to treat uterine leiomyosarcoma.
26. A computer-implemented method, wherein the method is as defined in any of aspects 1 to 17.
27. A computer comprising instructions for carrying out a method as defined in any of aspects 1 to 17.

The invention is described below by way of the following examples, which are merely illustrative and do not limit the scope of the invention.

### EXAMPLES

### Materials and Methods

### Clinical trial and study patient characteristics

A prospective, observational and multicenter case-control study was carried out to evaluate the diagnostic precision (sensitivity, specificity, negative predictive value and positive predictive value) in the detection of molecular differences by liquid biopsy in patients with a surgical indication for hysterectomy or myomectomy due to the diagnosis of myometrial tumors (leiomyoma / leiomyosarcoma) according to standard clinical practice.

Epidemiological, histopathological, and clinical outcomes of the 95 participant women between 18 and 80 years old and with a body mass index (BMI) of 18.5-40.0 kg/m², with suspected myometrial tumor (leiomyoma, LM / leiomyosarcoma, LMS) based on clinical symptoms and imaging diagnosis (LM, n = 72 and LMS, n = 23) were selected to participate in the present study. Women with the same age and BMI but without a uterine tumor were used as control subjects.

The objective of the study was to determine the molecular diagnosis of uterine tumors by liquid biopsy as a preoperative diagnosis, using pathological diagnosis as a "gold standard" to validate the molecular diagnosis.

Patients diagnosed with LM had a median age of 48 years (range: 30 - 71 years), while patients with LMS had a median age of 59 years (range: 43 - 82 years).

The use of human blood samples was previously approved by the Institutional Review Board of the 15 national hospitals involved in the study.

Patients signed and provided written informed consent. Those diagnosed with bacterial, fungal, or viral infections were excluded. This study was conducted in accordance with the Declaration of Helsinki. All specimens were anonymized after collection and histologically evaluated by expert pathologists to confirm the diagnosis according to World Health Organization criteria.

This study was registered on ClinicalTrials.gov with ID NCT04935333, and data were monitored by a clinical research associate.

### Clinical sample collection

After obtaining informed consent, 10-ml whole blood samples were collected from 97 patients with clinical diagnosis of myometrial tumor, and 25 control patients. All samples were processed in randomized batches. Briefly, one tube of blood was collected per patient in Cell-Free DNA BCTs (Streck Inc.) and processed into plasma. For this purpose, all blood was centrifuged at 1600g for 15 min, and plasma was transferred to a new tube which underwent a second centrifuge step. The plasma supernatant was aliquoted and stored at -80°C until use.

### Nucleic acid isolation, quality control and quantification

Ct-RNA was purified from plasma with the Serum/ Plasma Advanced Kit according to the manufacturer's instructions with minor modifications. Quality control and quantification analysis were performed using 2100 Bioanalyzer system (Agilent, Santa Clara, CA, USA). Samples with 100 ng total RNA were selected for further experiments.

### Ct-RNA library preparation and sequencing

Ct-RNA sequencing libraries were prepared using the Illumina RNA Prep (Illumina, California, USA), according to the manufacturer's instructions. In brief, biotinylated oligos targeting sequence-specific coding regions of the transcriptome were hybridized to sequencing libraries and pulled down by magnetic streptavidin beads to enrich libraries for RNA sequencing analysis. The final enriched library was then reamplified by PCR (manufacturer instructions, step 6th) to provide sufficient yield for the sequencing assay.

Control points for quantification of the intermediate and final c-DNA libraries were performed with DNA D1000 Analysis chips on the 2100 Bioanalyzer system (Agilent). Final enrichment libraries were quantified and normalized to a final library concentration of 10 nM through quantitative PCR assay (qPCR) using KAPA library Quant kit (Roche, Basilea, Switzerland) in a QuantStudio5 (Applied Biosystems, ThermoFisher Scientific, Waltham, Massachusetts). Lastly, each quantified library was pooled into a single pool with final concentration of ~2pM and loaded in a NextSeq 550.

### Bioinformatics analysis, data quality and filtering

Raw data from pair-ended Illumina sequencing was downloaded from Illumina BaseSpace and demultiplexed to generate FASTQ files using *bcl2fastq* conversion software (version 2.16.0.10) (https://emea.support.illumina.com/sequencing/sequencing_software/bcl2fastq-conversion-software.html). Then, each sample was aligned to the reference genome (GRCh37) using STAR alignment software (version 2.7.2). Uniquely mapped reads were filtered using *samtools* (version 1.9) and duplicates were removed with *PICARD* software (version 2.18.15) (https://broadinstitute.github.io/picard). Then, reads with mapping quality <10 were filtered using *samtools.* Finally, gene transcript abundance was estimated using the *HTseq-count* python package (version 0.11.2). In addition, duplicated reads rates were assessed using *dupRadar* R package. Its slope parameter indicates how fast duplicated reads increase in a sample.

### Differential gene expression analysis

Batch effect was corrected using Combat method included in SVA package (https://bioconductor.org/packages/sva). Differential gene expression analysis between LMS and LM samples was performed using the DEseq2 package from the Bioconductor repository and represented with ggplot2 (https://ggplot2.tidyverse.org) and EnhancedVolcano (https://bioconductor.org/packages/EnhancedVolcano) libraries. Data was normalized and features with a minimum of 10 counts in at least a number of samples were kept. The number of samples to apply the 10 counts threshold was calculated by the arithmetic mean of included samples divided by 2. Only genes with a |log2FC| is ≥ 2 and a p-Adjusted value < 0.05 were considered differentially expressed genes (DEGs).

### Example 1

A total of 440 genes were obtained from 5 selected group comparisons between leiomyosarcoma (LMS) and leiomyoma (LM) liquid biopsy samples (Figure 1).

Selected group comparisons differed in demographic and technical characteristics, such as the age of the subject, or the freezing time of the sample prior to the RNA extraction and data quality.

The 5 selected group comparisons were the following:
(a) *Comparison including all samples from the study*
*(b) Comparison including samples filtered by a quality threshold*
*(c) Comparison including samples which have been frozen for less than 6 months*
*(d) Comparison including samples which derive from subjects over 41 years* of *age*
(e) *Comparison including samples which have been frozen for less than 6 months and which derive from subjects over 41 years* of *age*

The complete list of the differentially expressed genes (DEGs) from the selected comparisons using liquid biopsy samples include 6 genes in common with the previous DEGs results of solid biopsy (WO2023061914) (Figure 2 and Table 1).

**Table 1: Common genes between solid and liquid biopsies results.**

| |
|---|
| POC1A |
| PTPRT |
| PTCHD1 |
| MUC4 |
| CENPM |
| KLHL41 |

### (a) Comparison including all samples from the study

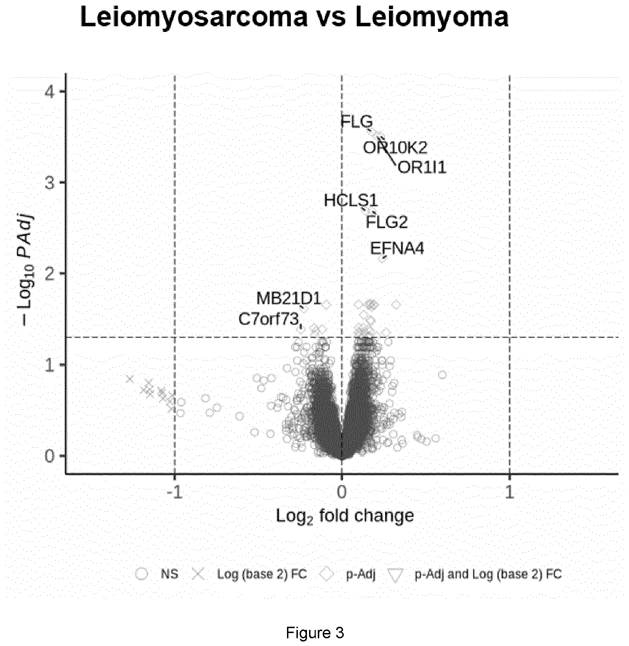

To identify DEGs in the comparison including all samples from the study, RNAseq analysis of 72 LM and 23 LMS tumor samples was performed. From this analysis, a total of 37 genes were found to be significantly differentially expressed between both groups of samples (LM and LMS), of which 30 genes were upregulated and 7 genes downregulated in LMS samples with respect to the reference value (Table 2 and Figure 3).

**Table 2: DEGs between LMS and LM from all samples comparison.**

| Gene | baseMean | log2FoldChange | IfcSE | stat | pvalue | padj |
|---|---|---|---|---|---|---|
| FLG | 1458.11569 | 0.17675 | 0.03122 | 5.66178 | 0 | 0.00028 |
| OR1I1 | 108.20656 | 0.21001 | 0.03844 | 5.4638 | 0 | 0.00031 |
| OR10K2 | 122.80325 | 0.22903 | 0.04203 | 5.44901 | 0 | 0.00031 |
| HCLS1 | 285.78447 | 0.14157 | 0.02815 | 5.02942 | 0 | 0.00205 |
| FLG2 | 1086.3725 | 0.17776 | 0.0355 | 5.00772 | 0 | 0.00205 |
| EFNA4 | 83.1639 | 0.24018 | 0.05074 | 4.73337 | 0 | 0.00685 |
| PKLR | 391.54662 | 0.16916 | 0.03789 | 4.4642 | 1E-05 | 0.02136 |
| ADAR | 747.30069 | 0.09892 | 0.02288 | 4.32278 | 2E-05 | 0.02205 |
| RPTN | 342.53078 | 0.15408 | 0.03516 | 4.38271 | 1E-05 | 0.02205 |
| OR9G4 | 122.52472 | 0.16789 | 0.03872 | 4.33658 | 1E-05 | 0.02205 |
| OR10T2 | 94.54094 | 0.1886 | 0.0433 | 4.3561 | 1E-05 | 0.02205 |
| PVRL4 | 293.48144 | 0.19026 | 0.0432 | 4.40444 | 1E-05 | 0.02205 |
| KRTAP6-1 | 34.12688 | 0.3237 | 0.07443 | 4.34885 | 1E-05 | 0.02205 |
| ARID5B | 407.01384 | -0.0937 | 0.02176 | -4.30613 | 2E-05 | 0.02208 |
| OR6K2 | 129.24063 | 0.17656 | 0.04126 | 4.27878 | 2E-05 | 0.02331 |
| MB21D1 | 119.79638 | -0.22914 | 0.05385 | -4.2551 | 2E-05 | 0.0243 |
| IGSF11 | 181.46701 | 0.12954 | 0.0308 | 4.20587 | 3E-05 | 0.02846 |
| OR1N1 | 84.21573 | 0.16927 | 0.04065 | 4.16373 | 3E-05 | 0.03236 |
| OR10J3 | 121.68788 | 0.17343 | 0.04184 | 4.1446 | 3E-05 | 0.03333 |
| RPS6 | 120.2214 | -0.16791 | 0.04102 | -4.09357 | 4E-05 | 0.03939 |
| EPHB1 | 411.94434 | 0.10026 | 0.02456 | 4.08258 | 4E-05 | 0.03939 |
| TNN | 500.62113 | 0.12546 | 0.03081 | 4.07217 | 5E-05 | 0.03939 |
| C7orf73 | 39.97076 | -0.24711 | 0.06105 | -4.04741 | 5E-05 | 0.04102 |
| KLF6 | 403.47339 | -0.11748 | 0.02907 | -4.04069 | 5E-05 | 0.04102 |
| NEUROD4 | 131.72981 | 0.15626 | 0.03875 | 4.0328 | 6E-05 | 0.04102 |
| SLX4IP | 190.38716 | -0.15683 | 0.03916 | -4.00514 | 6E-05 | 0.04271 |
| COA7 | 80.04748 | 0.16853 | 0.04207 | 4.00587 | 6E-05 | 0.04271 |
| ZNF333 | 336.82984 | 0.09638 | 0.0243 | 3.96596 | 7E-05 | 0.04447 |
| SMG5 | 467.07943 | 0.10928 | 0.02749 | 3.97477 | 7E-05 | 0.04447 |
| PLEK2 | 160.98117 | 0.13697 | 0.03456 | 3.96275 | 7E-05 | 0.04447 |
| BBS1 | 37.47083 | 0.26249 | 0.06619 | 3.96575 | 7E-05 | 0.04447 |
| HNRNPDL | 113.97844 | -0.164 | 0.0416 | -3.94259 | 8E-05 | 0.04686 |
| MUC1 | 179.57564 | 0.16248 | 0.04138 | 3.92612 | 9E-05 | 0.04867 |
| TTC21A | 595.56231 | 0.09918 | 0.02542 | 3.9016 | 0.0001 | 0.04906 |
| DNM2 | 699.94025 | 0.12592 | 0.0322 | 3.91121 | 9E-05 | 0.04906 |
| HRH1 | 143.954 | 0.15576 | 0.03997 | 3.89657 | 0.0001 | 0.04906 |
| GPR52 | 98.11404 | 0.207 | 0.05309 | 3.8987 | 0.0001 | 0.04906 |

### (b) Comparison including samples filtered by a quality threshold

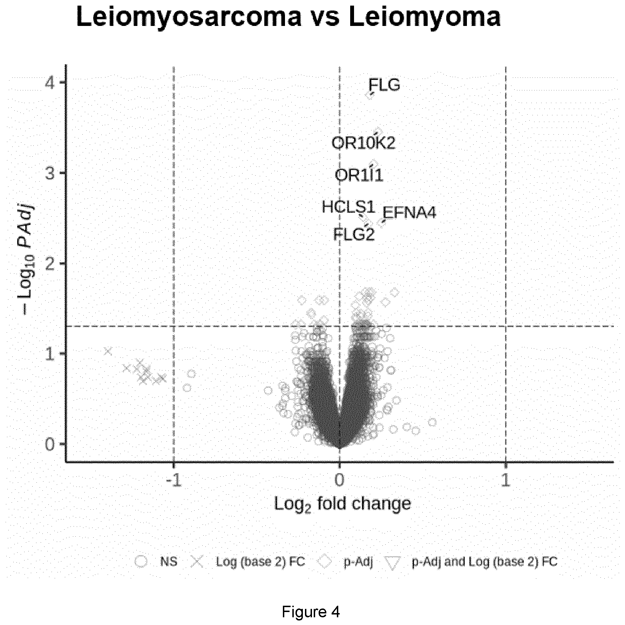

To identify DEGs in the comparison including samples filtered by a quality threshold, RNAseq analysis of 70 LM and 23 LMS tumor samples was performed. In this comparison, 2 LM samples, which had been frozen for more than 12 months, were filtered out due to having a dupRadar slope parameter < 8. This threshold allowed to maintain samples with a controlled level of duplicated reads while having a balanced number of samples and obtained results. From this analysis, a total of 55 genes were found to be significantly differentially expressed between both groups of samples, of which 44 genes were upregulated and 11 genes downregulated in LMS samples with respect to the reference value (Table 3 and Figure 4).

**Table 3: DEGs between LMS and LM from quality threshold comparison.**

| Gene | baseMean | log2FoldChange | IfcSE | stat | pvalue | padj |
|---|---|---|---|---|---|---|
| FLG | 1486.45103 | 0.18081 | 0.0313 | 5.77745 | 0 | 0.00014 |
| OR10K2 | 125.39435 | 0.23102 | 0.04203 | 5.4969 | 0 | 0.00035 |
| OR1I1 | 110.97664 | 0.20411 | 0.03868 | 5.27697 | 0 | 0.0008 |
| HCLS1 | 292.22579 | 0.14143 | 0.02847 | 4.96823 | 0 | 0.00308 |
| EFNA4 | 84.5096 | 0.25062 | 0.05119 | 4.89641 | 0 | 0.00356 |
| FLG2 | 1109.18896 | 0.17427 | 0.03588 | 4.85721 | 0 | 0.00362 |
| KRTAP6-1 | 34.90909 | 0.33044 | 0.07482 | 4.41649 | 1E-05 | 0.02088 |
| PVRL4 | 299.48744 | 0.19245 | 0.04386 | 4.38789 | 1E-05 | 0.02088 |
| OR6K2 | 132.0266 | 0.17736 | 0.04034 | 4.39702 | 1E-05 | 0.02088 |
| RPTN | 350.12285 | 0.15463 | 0.03484 | 4.43829 | 1E-05 | 0.02088 |
| SMG5 | 475.53875 | 0.11795 | 0.02715 | 4.34431 | 1E-05 | 0.02317 |
| OR10T2 | 96.75889 | 0.18667 | 0.0434 | 4.30132 | 2E-05 | 0.02383 |
| PKLR | 400.55186 | 0.16478 | 0.0382 | 4.31312 | 2E-05 | 0.02383 |
| OR1N1 | 85.88375 | 0.17228 | 0.04077 | 4.22594 | 2E-05 | 0.02554 |
| ARID5B | 415.46125 | -0.09341 | 0.02209 | -4.22857 | 2E-05 | 0.02554 |
| KLF6 | 413.3715 | -0.1231 | 0.02906 | -4.23566 | 2E-05 | 0.02554 |
| MB21D1 | 122.00429 | -0.229 | 0.05404 | -4.23773 | 2E-05 | 0.02554 |
| BBS1 | 37.64447 | 0.27572 | 0.06561 | 4.20243 | 3E-05 | 0.02677 |
| OR9G4 | 125.65474 | 0.16356 | 0.03907 | 4.18601 | 3E-05 | 0.02726 |
| ADAR | 764.70354 | 0.09534 | 0.02291 | 4.16102 | 3E-05 | 0.0289 |
| OR10J3 | 124.76643 | 0.17159 | 0.04196 | 4.08915 | 4E-05 | 0.0351 |
| EPHB1 | 420.8083 | 0.10116 | 0.02477 | 4.08409 | 4E-05 | 0.0351 |
| HNRNPDL | 117.08058 | -0.172 | 0.04193 | -4.10213 | 4E-05 | 0.0351 |
| IGSF11 | 185.32499 | 0.12804 | 0.03146 | 4.0697 | 5E-05 | 0.03579 |
| RPS6 | 122.80457 | -0.1705 | 0.04208 | -4.0521 | 5E-05 | 0.03705 |
| COA7 | 81.73413 | 0.17006 | 0.04224 | 4.02573 | 6E-05 | 0.03848 |
| TNN | 511.18487 | 0.12548 | 0.03117 | 4.02512 | 6E-05 | 0.03848 |
| FCRL5 | 515.62365 | 0.09775 | 0.02437 | 4.01059 | 6E-05 | 0.03947 |
| ZNF333 | 343.24612 | 0.09875 | 0.02474 | 3.99169 | 7E-05 | 0.04128 |
| PAX6 | 452.45009 | -0.09455 | 0.02379 | -3.97401 | 7E-05 | 0.04298 |
| B4GALT3 | 165.79708 | 0.15452 | 0.03896 | 3.96605 | 7E-05 | 0.04301 |
| GPR52 | 100.61509 | 0.20367 | 0.05318 | 3.83017 | 0.00013 | 0.04776 |
| ACER1 | 98.71037 | 0.16668 | 0.04335 | 3.84543 | 0.00012 | 0.04776 |
| FAM189B | 181.19829 | 0.16586 | 0.04325 | 3.83509 | 0.00013 | 0.04776 |
| MUC1 | 183.41554 | 0.16307 | 0.04203 | 3.87956 | 0.0001 | 0.04776 |
| SLC25A44 | 96.56203 | 0.16217 | 0.04229 | 3.83488 | 0.00013 | 0.04776 |
| OR5AR1 | 88.9408 | 0.15853 | 0.04134 | 3.83533 | 0.00013 | 0.04776 |
| DCST2 | 317.78392 | 0.15281 | 0.03995 | 3.82521 | 0.00013 | 0.04776 |
| DENND4B | 506.62558 | 0.15008 | 0.0384 | 3.90815 | 9E-05 | 0.04776 |
| NEUROD4 | 135.5637 | 0.1486 | 0.03836 | 3.87398 | 0.00011 | 0.04776 |
| OR5A2 | 124.06508 | 0.14697 | 0.03817 | 3.8507 | 0.00012 | 0.04776 |
| POC1A | 195.35978 | 0.14094 | 0.03685 | 3.82477 | 0.00013 | 0.04776 |
| PLEK2 | 164.19125 | 0.13606 | 0.03499 | 3.88912 | 0.0001 | 0.04776 |
| ATP8B2 | 512.12134 | 0.1299 | 0.03334 | 3.89668 | 0.0001 | 0.04776 |
| DNM2 | 715.45034 | 0.12734 | 0.03278 | 3.88465 | 0.0001 | 0.04776 |
| NCSTN | 684.53344 | 0.105 | 0.02739 | 3.83332 | 0.00013 | 0.04776 |
| LRRFIP1 | 549.51892 | -0.11605 | 0.03 | -3.86788 | 0.00011 | 0.04776 |
| SLX4IP | 194.16807 | -0.15342 | 0.03954 | -3.88024 | 0.0001 | 0.04776 |
| C7orf73 | 40.55219 | -0.235 | 0.06107 | -3.84802 | 0.00012 | 0.04776 |
| C4orf6 | 33.76979 | -0.26744 | 0.06931 | -3.85845 | 0.00011 | 0.04776 |
| FLVCR2 | 397.48692 | 0.11467 | 0.03006 | 3.81502 | 0.00014 | 0.04872 |
| ACKR2 | 110.62679 | 0.16615 | 0.0438 | 3.79337 | 0.00015 | 0.0493 |
| HHATL | 164.0402 | 0.15574 | 0.04095 | 3.80286 | 0.00014 | 0.0493 |
| PTPRT | 650.11231 | 0.0977 | 0.02573 | 3.79668 | 0.00015 | 0.0493 |
| NUP50 | 182.27577 | -0.13595 | 0.03577 | -3.80026 | 0.00014 | 0.0493 |

### (c) Comparison including samples which have been frozen for less than 6 months

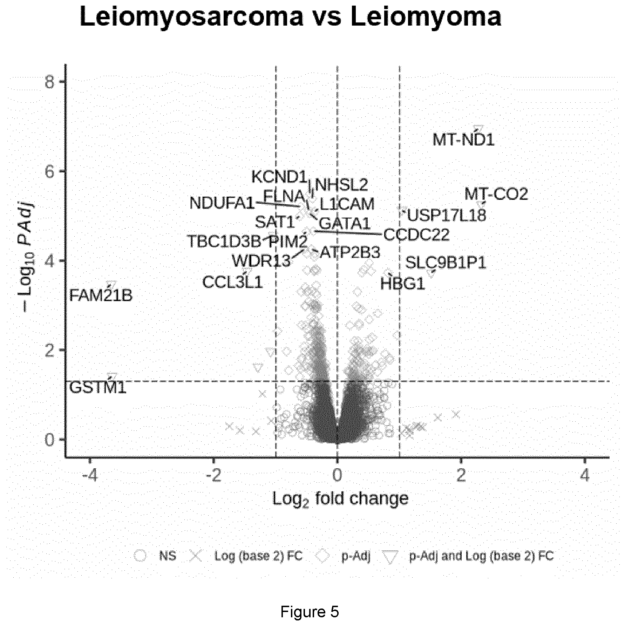

To identify DEGs in this comparison, RNAseq analysis of 19 LM and 7 LMS tumor samples which have been frozen for less than 6 months was performed. From this analysis, a total of 378 genes were found to be significantly differentially expressed between both groups of samples, of which 100 genes were upregulated and 278 genes downregulated in LMS samples with respect to the reference value (Tables 4 and 5 and Figure 5).

**Table 4: DEGs between LMS and LM samples which have been frozen for less than 6 months (columns are separated by commas).**

| Gene | baseMean | log2FoldChange | lfcSE | stat | pvalue | padj |
|---|---|---|---|---|---|---|
| MT-ND1 | 31.15932 | 2.27773 | 0.33108 | 6.87971 | 0 | 0 |
| NHSL2 | 337.76049 | -0.40681 | 0.06588 | -6.17514 | 0 | 0 |
| KCND1 | 211.34632 | -0.44795 | 0.07135 | -6.27848 | 0 | 0 |
| MT-CO2 | 20.2197 | 2.32409 | 0.3821 | 6.08243 | 0 | 1E-05 |
| USP17L18 | 32.45514 | 1.03721 | 0.1745 | 5.94386 | 0 | 1E-05 |
| L1CAM | 792.67814 | -0.37129 | 0.06282 | -5.90989 | 0 | 1E-05 |
| GATA1 | 187.41502 | -0.45192 | 0.07694 | -5.87371 | 0 | 1E-05 |
| FLNA | 1170.72723 | -0.46802 | 0.07866 | -5.94963 | 0 | 1E-05 |
| NDUFA1 | 90.11203 | -0.59598 | 0.09886 | -6.02849 | 0 | 1E-05 |
| SAT1 | 81.5517 | -0.61186 | 0.10488 | -5.83369 | 0 | 1E-05 |
| CCDC22 | 242.97481 | -0.38898 | 0.06839 | -5.68773 | 0 | 2E-05 |
| PIM2 | 104.4135 | -0.5004 | 0.08828 | -5.66829 | 0 | 2E-05 |
| TBC1 D3B | 70.99607 | -1.04502 | 0.18586 | -5.62258 | 0 | 3E-05 |
| ATP2B3 | 468.8959 | -0.42226 | 0.07705 | -5.4802 | 0 | 5E-05 |
| WDR13 | 127.62145 | -0.5348 | 0.09771 | -5.47318 | 0 | 5E-05 |
| STARD8 | 384.48379 | -0.36572 | 0.06762 | -5.40855 | 0 | 7E-05 |
| FAM155B | 105.20249 | -0.50006 | 0.09239 | -5.41253 | 0 | 7E-05 |
| GRIPAP1 | 409.66654 | -0.37359 | 0.0696 | -5.36783 | 0 | 8E-05 |
| MBD3L1 | 53.37601 | 0.51579 | 0.0974 | 5.29531 | 0 | 0.00011 |
| TCEAL1 | 31.12892 | -0.78846 | 0.14841 | -5.31278 | 0 | 0.00011 |
| BCORL1 | 548.42461 | -0.35042 | 0.06703 | -5.22815 | 0 | 0.00015 |
| OR2J1 | 85.61589 | 0.42436 | 0.08139 | 5.21383 | 0 | 0.00016 |
| IQSEC2 | 557.66463 | -0.33105 | 0.06388 | -5.18265 | 0 | 0.00016 |
| PRPS2 | 150.19095 | -0.37751 | 0.07263 | -5.19782 | 0 | 0.00016 |
| TSPAN7 | 99.39778 | -0.49277 | 0.0951 | -5.18158 | 0 | 0.00016 |
| KRTAP10-6 | 57.12107 | 0.54312 | 0.10531 | 5.15738 | 0 | 0.00017 |
| TEX11 | 544.69999 | -0.37859 | 0.07347 | -5.15308 | 0 | 0.00017 |
| CCL3L1 | 15.84417 | -1.46076 | 0.2829 | -5.16349 | 0 | 0.00017 |
| SLC9B1P1 | 8.33748 | 1.51273 | 0.29539 | 5.12112 | 0 | 0.00018 |
| HBG1 | 25.73016 | 0.8114 | 0.15853 | 5.11828 | 0 | 0.00018 |
| C16orf52 | 134.12027 | 0.39724 | 0.07759 | 5.1198 | 0 | 0.00018 |
| FAM21B | 110.70743 | -3.65654 | 0.73151 | -4.9986 | 0 | 0.00034 |
| PTCHD1 | 355.14199 | -0.34612 | 0.06946 | -4.98284 | 0 | 0.00035 |
| PRRG3 | 93.03466 | -0.52191 | 0.1053 | -4.95653 | 0 | 0.00039 |
| AVPR2 | 134.58496 | -0.41524 | 0.08463 | -4.9067 | 0 | 0.00048 |
| CXorf64 | 93.48364 | -0.47963 | 0.09771 | -4.90843 | 0 | 0.00048 |
| PSG3 | 186.84283 | 0.32492 | 0.06637 | 4.89534 | 0 | 0.00049 |
| CT55 | 80.64772 | -0.49538 | 0.10142 | -4.88452 | 0 | 0.0005 |
| TSC22D3 | 88.63042 | -0.58805 | 0.1204 | -4.88405 | 0 | 0.0005 |
| SRPX | 181.96947 | -0.35681 | 0.07328 | -4.86921 | 0 | 0.00052 |
| WDR44 | 454.02164 | -0.33615 | 0.06943 | -4.84148 | 0 | 0.00058 |
| SUV39H1 | 76.46488 | -0.46329 | 0.09707 | -4.77256 | 0 | 0.0008 |
| RNF128 | 205.15648 | -0.37191 | 0.0786 | -4.73186 | 0 | 0.00093 |
| CFP | 212.33056 | -0.38891 | 0.08213 | -4.73511 | 0 | 0.00093 |
| AWAT2 | 162.88806 | -0.43005 | 0.09095 | -4.72843 | 0 | 0.00093 |
| LUZP4 | 175.00844 | -0.35024 | 0.07424 | -4.71754 | 0 | 0.00096 |
| MAOA | 567.72199 | -0.31212 | 0.06623 | -4.7128 | 0 | 0.00097 |
| FTSJ 1 | 282.07029 | -0.36743 | 0.07811 | -4.70413 | 0 | 0.00099 |
| PRAMEF2 | 186.03488 | -0.52581 | 0.112 | -4.69455 | 0 | 0.00101 |
| PRKX | 148.47189 | -0.34474 | 0.07353 | -4.68814 | 0 | 0.00102 |
| PSG2 | 188.71953 | 0.30875 | 0.06592 | 4.68355 | 0 | 0.00103 |
| RHOXF1 | 49.04325 | -0.51474 | 0.11006 | -4.67696 | 0 | 0.00104 |
| DOCK11 | 1049.67276 | -0.33619 | 0.072 | -4.66921 | 0 | 0.00106 |
| TRIM65 | 116.05266 | 0.34264 | 0.07381 | 4.64199 | 0 | 0.00118 |
| FGD1 | 516.45246 | -0.33235 | 0.07164 | -4.6394 | 0 | 0.00118 |
| TAZ | 293.00777 | -0.33903 | 0.07318 | -4.63289 | 0 | 0.0012 |
| HAUS1 | 141.28082 | -0.3649 | 0.07893 | -4.62293 | 0 | 0.00121 |
| OTC | 385.31681 | -0.37223 | 0.08046 | -4.62643 | 0 | 0.00121 |
| FAM211A | 78.53169 | 0.39232 | 0.08506 | 4.61216 | 0 | 0.00125 |
| RNASE13 | 23.66783 | 0.64827 | 0.14185 | 4.57023 | 0 | 0.00151 |
| FRMPD3 | 581.40043 | -0.30458 | 0.06713 | -4.53716 | 1E-05 | 0.00174 |
| FUNDC1 | 84.09754 | -0.42788 | 0.09475 | -4.51573 | 1E-05 | 0.00189 |
| SMS | 369.13328 | -0.36753 | 0.08177 | -4.49476 | 1E-05 | 0.00205 |
| OPA3 | 63.25833 | 0.51418 | 0.11459 | 4.48715 | 1E-05 | 0.0021 |
| PSG5 | 182.68956 | 0.35216 | 0.07933 | 4.439 | 1E-05 | 0.00256 |
| GPKOW | 185.45024 | -0.38642 | 0.08707 | -4.43803 | 1E-05 | 0.00256 |
| RBMX2 | 114.65648 | -0.38566 | 0.08719 | -4.42325 | 1E-05 | 0.00266 |
| RPL10 | 156.42212 | -0.39627 | 0.0896 | -4.42259 | 1E-05 | 0.00266 |
| NDUFB11 | 44.03605 | -0.58175 | 0.13194 | -4.40926 | 1E-05 | 0.00279 |
| GPR52 | 100.35347 | 0.34859 | 0.07929 | 4.39663 | 1E-05 | 0.00284 |
| FLG2 | 1137.30557 | 0.19984 | 0.0454 | 4.40154 | 1E-05 | 0.00284 |
| CXorf40B | 69.98453 | -0.51601 | 0.11734 | -4.39755 | 1E-05 | 0.00284 |
| TKTL1 | 286.57103 | -0.29864 | 0.06803 | -4.39001 | 1E-05 | 0.00288 |
| FAM90A1 | 367.24366 | -0.3263 | 0.07454 | -4.37743 | 1E-05 | 0.00301 |
| GJB1 | 77.27936 | -0.49687 | 0.11432 | -4.3462 | 1E-05 | 0.00343 |
| ZFX | 429.90822 | -0.28213 | 0.06517 | -4.32885 | 1E-05 | 0.00362 |
| PIR | 209.34383 | -0.34033 | 0.07859 | -4.33023 | 1E-05 | 0.00362 |
| SRPK3 | 171.10616 | -0.31367 | 0.07252 | -4.32528 | 2E-05 | 0.00363 |
| MB21D1 | 119.56685 | -0.34487 | 0.07987 | -4.3178 | 2E-05 | 0.00371 |
| HSDL2 | 182.44055 | -0.2595 | 0.06036 | -4.29922 | 2E-05 | 0.00379 |
| ARRDC4 | 174.86941 | -0.28429 | 0.06612 | -4.29963 | 2E-05 | 0.00379 |
| FOXO4 | 168.48099 | -0.31798 | 0.07386 | -4.30509 | 2E-05 | 0.00379 |
| HMGN5 | 119.86672 | -0.37914 | 0.08816 | -4.30086 | 2E-05 | 0.00379 |
| FCGR3B | 49.10344 | -0.97265 | 0.22615 | -4.30096 | 2E-05 | 0.00379 |
| CD40LG | 216.34862 | -0.35608 | 0.08289 | -4.29589 | 2E-05 | 0.0038 |
| BEST4 | 94.35534 | -0.38034 | 0.08907 | -4.27031 | 2E-05 | 0.00422 |
| MCF2 | 602.5511 | -0.29979 | 0.07065 | -4.24358 | 2E-05 | 0.0047 |
| SCAND1 | 10.64838 | 0.96235 | 0.2271 | 4.23751 | 2E-05 | 0.00472 |
| FAM122B | 178.09847 | -0.32263 | 0.07614 | -4.23734 | 2E-05 | 0.00472 |
| CHDC2 | 256.37368 | -0.35604 | 0.08423 | -4.22671 | 2E-05 | 0.00487 |
| OTUD6A | 62.70451 | -0.45371 | 0.10741 | -4.22404 | 2E-05 | 0.00487 |
| NPIPB6 | 102.92334 | -0.4796 | 0.11357 | -4.22296 | 2E-05 | 0.00487 |
| ACE2 | 392.32564 | -0.29491 | 0.06993 | -4.21727 | 2E-05 | 0.00494 |
| NONO | 209.60103 | -0.28368 | 0.0674 | -4.20886 | 3E-05 | 0.00507 |
| HLA-A | 59.40584 | 0.56031 | 0.13346 | 4.19846 | 3E-05 | 0.00526 |
| ELF4 | 351.75627 | -0.27377 | 0.0654 | -4.18595 | 3E-05 | 0.0055 |
| JOSD2 | 43.59233 | 0.51669 | 0.12392 | 4.16962 | 3E-05 | 0.00584 |
| CXorf22 | 380.58417 | -0.30403 | 0.07317 | -4.15524 | 3E-05 | 0.0061 |
| PNMA3 | 170.83486 | -0.33728 | 0.08114 | -4.15688 | 3E-05 | 0.0061 |
| PCDHA4 | 152.25338 | 0.27559 | 0.06649 | 4.14507 | 3E-05 | 0.00631 |
| UBQLN2 | 197.07479 | -0.31621 | 0.07635 | -4.14183 | 3E-05 | 0.00634 |
| CHRNA10 | 102.73351 | 0.33223 | 0.08044 | 4.13042 | 4E-05 | 0.00635 |
| TLR8 | 298.54575 | -0.26022 | 0.06299 | -4.13086 | 4E-05 | 0.00635 |
| POLA1 | 798.70115 | -0.27622 | 0.06685 | -4.13187 | 4E-05 | 0.00635 |
| TBC1D8B | 508.78564 | -0.34436 | 0.08332 | -4.13272 | 4E-05 | 0.00635 |
| TIMM8A | 78.9981 | -0.4317 | 0.10436 | -4.1366 | 4E-05 | 0.00635 |
| IDH3G | 189.60997 | -0.31252 | 0.07623 | -4.09961 | 4E-05 | 0.00719 |
| VSIG1 | 190.39869 | -0.29688 | 0.07262 | -4.08823 | 4E-05 | 0.00748 |
| APOO | 159.79247 | -0.35381 | 0.08668 | -4.0817 | 4E-05 | 0.00755 |
| FAM3A | 161.58332 | -0.35877 | 0.08785 | -4.08372 | 4E-05 | 0.00755 |
| DMD | 3102.43735 | -0.26545 | 0.06514 | -4.07479 | 5E-05 | 0.00764 |
| BOLL | 185.27323 | -0.27445 | 0.06734 | -4.07555 | 5E-05 | 0.00764 |
| TAGLN2 | 92.58855 | 0.33704 | 0.08301 | 4.06008 | 5E-05 | 0.00804 |
| NAP1L3 | 135.56791 | -0.34169 | 0.08418 | -4.05878 | 5E-05 | 0.00804 |
| HS6ST2 | 150.68337 | -0.3094 | 0.07627 | -4.0564 | 5E-05 | 0.00805 |
| TXLNG | 204.7201 | -0.27142 | 0.06704 | -4.04897 | 5E-05 | 0.00824 |
| BRCC3 | 187.90731 | -0.30953 | 0.07653 | -4.04444 | 5E-05 | 0.00833 |
| DNASE1L1 | 48.57917 | -0.54118 | 0.13389 | -4.0419 | 5E-05 | 0.00835 |
| FAM120C | 451.23236 | -0.25111 | 0.06224 | -4.03436 | 5E-05 | 0.0085 |
| TMEM187 | 76.30269 | -0.40937 | 0.10149 | -4.03375 | 5E-05 | 0.0085 |
| ZNF280C | 367.0461 | -0.33148 | 0.08248 | -4.019 | 6E-05 | 0.00897 |
| ZBTB33 | 193.67958 | -0.29047 | 0.07236 | -4.01417 | 6E-05 | 0.00901 |
| FAM47A | 192.42404 | -0.30922 | 0.077 | -4.01605 | 6E-05 | 0.00901 |
| PASD1 | 276.20554 | -0.32567 | 0.08117 | -4.01233 | 6E-05 | 0.00901 |
| ZNF547 | 149.01798 | 0.27431 | 0.06845 | 4.00742 | 6E-05 | 0.00912 |
| HLA-DOB | 34.99197 | 0.48115 | 0.12026 | 4.00104 | 6E-05 | 0.0093 |
| LRP5L | 153.06081 | 0.33529 | 0.08393 | 3.99506 | 6E-05 | 0.00939 |
| OCRL | 852.74224 | -0.2463 | 0.06163 | -3.99649 | 6E-05 | 0.00939 |
| DCAF12L2 | 83.68357 | -0.375 | 0.09438 | -3.97346 | 7E-05 | 0.0102 |
| B3GNT6 | 29.20879 | 0.57423 | 0.14468 | 3.96894 | 7E-05 | 0.01027 |
| OR8H2 | 87.63052 | 0.35233 | 0.08879 | 3.96808 | 7E-05 | 0.01027 |
| HCCS | 234.45876 | -0.29554 | 0.07459 | -3.9623 | 7E-05 | 0.01037 |
| MAGEC2 | 96.64483 | -0.35032 | 0.08839 | -3.96349 | 7E-05 | 0.01037 |
| PLS3 | 376.70162 | -0.34338 | 0.08678 | -3.95701 | 8E-05 | 0.01052 |
| ARSH | 229.72064 | -0.29113 | 0.07361 | -3.95498 | 8E-05 | 0.01053 |
| POTEC | 308.25265 | -0.26454 | 0.06694 | -3.95204 | 8E-05 | 0.01058 |
| TANGO2 | 127.81251 | 0.33184 | 0.08405 | 3.94823 | 8E-05 | 0.0106 |
| HDAC6 | 520.37296 | -0.24657 | 0.06245 | -3.94809 | 8E-05 | 0.0106 |
| FAM47C | 286.29155 | -0.29443 | 0.0746 | -3.94654 | 8E-05 | 0.0106 |
| CYBB | 548.05348 | -0.28951 | 0.0734 | -3.94439 | 8E-05 | 0.01062 |
| DUSP9 | 63.02302 | -0.46454 | 0.11789 | -3.94065 | 8E-05 | 0.01071 |
| KRTAP5-10 | 38.37645 | 0.52294 | 0.133 | 3.93186 | 8E-05 | 0.01078 |
| SLIT1 | 33.6224 | 0.48969 | 0.12459 | 3.93043 | 8E-05 | 0.01078 |
| MUC4 | 891.52779 | 0.246 | 0.06261 | 3.92903 | 9E-05 | 0.01078 |
| DCTN3 | 140.5943 | -0.28341 | 0.07206 | -3.93292 | 8E-05 | 0.01078 |
| IRAK1 | 207.60107 | -0.33158 | 0.08446 | -3.92569 | 9E-05 | 0.01078 |
| ATOH1 | 35.92665 | -0.53155 | 0.13537 | -3.92655 | 9E-05 | 0.01078 |
| PRR23D1 | 25.22845 | -0.82631 | 0.21012 | -3.93253 | 8E-05 | 0.01078 |
| C3orf80 | 10.22808 | -1.08434 | 0.27583 | -3.9312 | 8E-05 | 0.01078 |
| C20orf202 | 28.62355 | 0.55634 | 0.14178 | 3.92391 | 9E-05 | 0.01079 |
| RPS3A | 56.49118 | -0.40387 | 0.10298 | -3.92177 | 9E-05 | 0.01081 |
| TBC1D28 | 85.75607 | 0.37734 | 0.09628 | 3.91932 | 9E-05 | 0.01085 |
| SLC16A8 | 54.10367 | 0.45662 | 0.11659 | 3.91655 | 9E-05 | 0.01091 |
| GAB3 | 221.98657 | -0.29076 | 0.07437 | -3.90954 | 9E-05 | 0.01116 |
| CXorf67 | 86.87845 | -0.38892 | 0.09959 | -3.90517 | 9E-05 | 0.01129 |
| SLC9A6 | 544.08375 | -0.29315 | 0.07525 | -3.89546 | 0.0001 | 0.01165 |
| TFF2 | 64.0809 | -0.38613 | 0.09915 | -3.89435 | 0.0001 | 0.01165 |
| CTPS2 | 377.91465 | -0.26521 | 0.0682 | -3.88867 | 0.0001 | 0.01185 |
| LZIC | 120.42089 | 0.2943 | 0.07572 | 3.88674 | 0.0001 | 0.01187 |
| PHKA1 | 1251.69142 | -0.28134 | 0.07248 | -3.88157 | 0.0001 | 0.01205 |
| CNKSR2 | 545.66536 | -0.26936 | 0.06945 | -3.87833 | 0.00011 | 0.01214 |
| C4orf48 | 12.60875 | 0.76577 | 0.1976 | 3.87527 | 0.00011 | 0.01218 |
| OR10H3 | 90.29974 | 0.29124 | 0.07517 | 3.87444 | 0.00011 | 0.01218 |
| HCN2 | 107.68994 | -0.30764 | 0.07957 | -3.86629 | 0.00011 | 0.01252 |
| FAM230A | 47.02168 | 0.5067 | 0.13117 | 3.86298 | 0.00011 | 0.01254 |
| ATP11C | 633.41774 | -0.27744 | 0.07182 | -3.86314 | 0.00011 | 0.01254 |
| FKBP1A | 50.33631 | 0.39117 | 0.10149 | 3.85414 | 0.00012 | 0.01292 |
| CXorf57 | 324.28556 | -0.27903 | 0.07248 | -3.84989 | 0.00012 | 0.01299 |
| PPP3R2 | 54.0519 | -0.39895 | 0.10359 | -3.85141 | 0.00012 | 0.01299 |
| HTATSF1 | 268.37687 | -0.26885 | 0.06988 | -3.84756 | 0.00012 | 0.01304 |
| GDI1 | 328.07843 | -0.29312 | 0.07622 | -3.84571 | 0.00012 | 0.01306 |
| TMSB15A | 47.10065 | -0.47115 | 0.12288 | -3.83429 | 0.00013 | 0.0136 |
| KLF8 | 167.08752 | -0.27271 | 0.07117 | -3.83181 | 0.00013 | 0.01366 |
| ITIH6 | 432.94886 | -0.25355 | 0.06651 | -3.81193 | 0.00014 | 0.01464 |
| PRDX4 | 170.45656 | -0.29997 | 0.07867 | -3.8132 | 0.00014 | 0.01464 |
| UBE2NL | 58.22869 | -0.38303 | 0.1007 | -3.80384 | 0.00014 | 0.01495 |
| HIST1H2AB | 30.16036 | -0.57432 | 0.15095 | -3.80465 | 0.00014 | 0.01495 |
| IL17D | 8.90737 | 0.8337 | 0.21945 | 3.79913 | 0.00015 | 0.01507 |
| FMR1NB | 118.06902 | -0.3119 | 0.08208 | -3.79973 | 0.00014 | 0.01507 |
| FAM212B | 104.83328 | 0.31441 | 0.08289 | 3.79325 | 0.00015 | 0.01534 |
| RGAG4 | 119.86271 | -0.3434 | 0.09056 | -3.79196 | 0.00015 | 0.01534 |
| PORCN | 414.24019 | -0.26703 | 0.07055 | -3.78481 | 0.00015 | 0.01565 |
| CBLN1 | 68.87156 | -0.35455 | 0.09369 | -3.78427 | 0.00015 | 0.01565 |
| ZNF671 | 144.13076 | 0.24467 | 0.0647 | 3.7814 | 0.00016 | 0.01574 |
| NUDT11 | 37.06305 | -0.47562 | 0.12608 | -3.7723 | 0.00016 | 0.01624 |
| KDM6A | 1042.13898 | -0.25846 | 0.06857 | -3.76905 | 0.00016 | 0.01637 |
| ARHGAP4 | 226.96162 | -0.30479 | 0.08097 | -3.76439 | 0.00017 | 0.01641 |
| CYLC1 | 203.56831 | -0.35275 | 0.09367 | -3.76573 | 0.00017 | 0.01641 |
| OCLM | 26.92075 | -0.58781 | 0.15615 | -3.76442 | 0.00017 | 0.01641 |
| TFDP3 | 124.59399 | -0.2918 | 0.07757 | -3.76165 | 0.00017 | 0.0165 |
| SDHAF1 | 8.98845 | 0.84486 | 0.2249 | 3.75661 | 0.00017 | 0.01675 |
| ZNF81 | 367.53342 | -0.27654 | 0.07367 | -3.75378 | 0.00017 | 0.01685 |
| GLA | 323.08751 | -0.25584 | 0.06818 | -3.75225 | 0.00018 | 0.01687 |
| C7orf71 | 76.51124 | 0.31415 | 0.08381 | 3.74822 | 0.00018 | 0.01705 |
| GUK1 | 95.77112 | 0.3443 | 0.09193 | 3.74521 | 0.00018 | 0.01717 |
| FAM217B | 107.90028 | 0.27462 | 0.07358 | 3.73228 | 0.00019 | 0.01799 |
| ZNF645 | 130.87937 | -0.31345 | 0.08415 | -3.72483 | 0.0002 | 0.01834 |
| TMEM27 | 119.25303 | -0.3194 | 0.08574 | -3.72512 | 0.0002 | 0.01834 |
| C19orf35 | 20.03834 | 0.70818 | 0.19054 | 3.71679 | 0.0002 | 0.01884 |
| LACTBL1 | 67.9708 | 0.37251 | 0.10041 | 3.70974 | 0.00021 | 0.01927 |
| OR52B6 | 93.94985 | 0.31259 | 0.08439 | 3.70417 | 0.00021 | 0.0196 |
| KCNJ4 | 42.70677 | 0.43827 | 0.11847 | 3.69944 | 0.00022 | 0.01977 |
| CXorf23 | 321.15698 | -0.28325 | 0.07654 | -3.70051 | 0.00022 | 0.01977 |
| GK | 602.80958 | -0.30494 | 0.08249 | -3.69687 | 0.00022 | 0.01988 |
| LONRF2 | 263.19324 | -0.1885 | 0.05106 | -3.69213 | 0.00022 | 0.02015 |
| PAK3 | 555.40271 | -0.27034 | 0.07326 | -3.6902 | 0.00022 | 0.02021 |
| UXT | 69.8148 | -0.3954 | 0.10721 | -3.68819 | 0.00023 | 0.02027 |
| HDAC8 | 358.75452 | -0.22329 | 0.06066 | -3.68082 | 0.00023 | 0.02071 |
| LRCH2 | 318.12773 | -0.30158 | 0.08195 | -3.68021 | 0.00023 | 0.02071 |
| ADIPOR1 | 145.26862 | -0.28067 | 0.0763 | -3.67837 | 0.00023 | 0.02076 |
| RNF185 | 122.86874 | 0.24629 | 0.06698 | 3.67714 | 0.00024 | 0.02077 |
| AMER1 | 371.98414 | -0.2572 | 0.07008 | -3.67032 | 0.00024 | 0.02113 |
| LHFPL1 | 91.01003 | -0.35718 | 0.09729 | -3.67134 | 0.00024 | 0.02113 |
| EMD | 105.23142 | -0.37158 | 0.1015 | -3.66102 | 0.00025 | 0.02181 |
| HIST1H3H | 23.94268 | 0.61392 | 0.16786 | 3.65733 | 0.00025 | 0.02182 |
| CACNA1F | 1361.07181 | -0.2496 | 0.06824 | -3.65759 | 0.00025 | 0.02182 |
| CENPM | 99.80098 | -0.3039 | 0.08309 | -3.65742 | 0.00025 | 0.02182 |
| CACNG5 | 123.90538 | -0.28573 | 0.07824 | -3.65217 | 0.00026 | 0.02216 |
| FAM209A | 37.30314 | 0.4348 | 0.11927 | 3.64554 | 0.00027 | 0.02233 |
| ZBED6 | 105.82195 | 0.26891 | 0.07374 | 3.64697 | 0.00027 | 0.02233 |
| OFD1 | 796.43072 | -0.28002 | 0.07676 | -3.64789 | 0.00026 | 0.02233 |
| CTAGE6 | 95.24419 | -0.63045 | 0.1729 | -3.64633 | 0.00027 | 0.02233 |
| KRTAP4-4 | 20.28861 | 0.71923 | 0.19755 | 3.64077 | 0.00027 | 0.02249 |
| OR7D4 | 130.33322 | 0.25333 | 0.06956 | 3.64169 | 0.00027 | 0.02249 |
| P2RY4 | 88.48628 | -0.36282 | 0.09967 | -3.64024 | 0.00027 | 0.02249 |
| LONRF3 | 305.9904 | -0.21714 | 0.0597 | -3.63698 | 0.00028 | 0.02262 |
| LAS1L | 299.76639 | -0.28069 | 0.07719 | -3.63652 | 0.00028 | 0.02262 |
| RFC5 | 222.47678 | -0.20872 | 0.05742 | -3.63509 | 0.00028 | 0.02264 |
| F8 | 1411.05276 | -0.25239 | 0.06948 | -3.63249 | 0.00028 | 0.02268 |
| FGL1 | 129.42962 | -0.28142 | 0.07747 | -3.6324 | 0.00028 | 0.02268 |
| FAM104B | 89.0675 | -0.33092 | 0.09114 | -3.63092 | 0.00028 | 0.02271 |
| FAM133A | 76.19086 | -0.34783 | 0.09588 | -3.62764 | 0.00029 | 0.0229 |
| MRPL2 | 109.53556 | -0.29336 | 0.08108 | -3.61803 | 0.0003 | 0.02367 |
| OPN1MW | 29.36525 | -1.29013 | 0.35674 | -3.61642 | 0.0003 | 0.02371 |
| CTAG2 | 54.95942 | -0.53765 | 0.14875 | -3.61437 | 0.0003 | 0.0238 |
| CAPN6 | 290.01204 | -0.26928 | 0.07462 | -3.60848 | 0.00031 | 0.02414 |
| SPAG11B | 100.79713 | -0.48594 | 0.13465 | -3.60885 | 0.00031 | 0.02414 |
| C1orf210 | 38.84506 | 0.42079 | 0.11673 | 3.60475 | 0.00031 | 0.02439 |
| CENPI | 399.50202 | -0.293 | 0.08134 | -3.60234 | 0.00032 | 0.02441 |
| PPP1R14A | 42.52071 | -0.43019 | 0.11939 | -3.60317 | 0.00031 | 0.02441 |
| EDA | 285.36926 | -0.2808 | 0.07799 | -3.6003 | 0.00032 | 0.0245 |
| NAA10 | 78.89488 | -0.32551 | 0.09046 | -3.59856 | 0.00032 | 0.02456 |
| C10orf82 | 107.27524 | -0.27174 | 0.07557 | -3.59603 | 0.00032 | 0.0247 |
| G6PD | 261.74516 | -0.29438 | 0.08206 | -3.58729 | 0.00033 | 0.02544 |
| CYSLTR1 | 112.09995 | -0.32729 | 0.09128 | -3.58551 | 0.00034 | 0.02551 |
| TLR7 | 355.00292 | -0.24431 | 0.06818 | -3.58323 | 0.00034 | 0.02552 |
| ZNF275 | 130.08923 | -0.39891 | 0.11129 | -3.58428 | 0.00034 | 0.02552 |
| GREM2 | 37.68735 | 0.48373 | 0.13505 | 3.5818 | 0.00034 | 0.02556 |
| SSB | 198.49983 | -0.24917 | 0.06965 | -3.57744 | 0.00035 | 0.02571 |
| IL1RAPL2 | 291.20518 | -0.26481 | 0.07402 | -3.5777 | 0.00035 | 0.02571 |
| FAM199X | 150.53219 | -0.26929 | 0.07528 | -3.5771 | 0.00035 | 0.02571 |
| TCEAL4 | 132.47625 | -0.26565 | 0.07433 | -3.57396 | 0.00035 | 0.02573 |
| CUL4B | 676.23351 | -0.28488 | 0.07971 | -3.57383 | 0.00035 | 0.02573 |
| GBX1 | 83.41772 | -0.32222 | 0.09014 | -3.57482 | 0.00035 | 0.02573 |
| PHF6 | 283.56016 | -0.2935 | 0.08217 | -3.57186 | 0.00035 | 0.02582 |
| BRWD3 | 1347.59232 | -0.26713 | 0.07484 | -3.56961 | 0.00036 | 0.02594 |
| PTDSS2 | 179.08695 | 0.24543 | 0.06878 | 3.56852 | 0.00036 | 0.02595 |
| SLITRK4 | 265.14823 | -0.25845 | 0.07245 | -3.56726 | 0.00036 | 0.02597 |
| RAB42 | 20.66735 | 0.53975 | 0.15152 | 3.56213 | 0.00037 | 0.02617 |
| EDA2R | 136.99279 | -0.27315 | 0.07668 | -3.56197 | 0.00037 | 0.02617 |
| RPS6KA6 | 395.85404 | -0.30337 | 0.08516 | -3.56233 | 0.00037 | 0.02617 |
| MAGEB6 | 97.44217 | -0.32787 | 0.09207 | -3.56129 | 0.00037 | 0.02617 |
| LRRC27 | 311.44364 | -0.19232 | 0.05403 | -3.55979 | 0.00037 | 0.02622 |
| SEC16B | 20.70249 | -0.59535 | 0.1673 | -3.55854 | 0.00037 | 0.02624 |
| POF1B | 311.51465 | -0.30092 | 0.08475 | -3.5506 | 0.00038 | 0.02694 |
| SCD | 170.12531 | -0.22907 | 0.06459 | -3.54669 | 0.00039 | 0.02724 |
| ZNF75D | 246.57283 | -0.26213 | 0.07393 | -3.54565 | 0.00039 | 0.02725 |
| CNNM1 | 254.32335 | -0.19753 | 0.05573 | -3.54426 | 0.00039 | 0.02729 |
| OR4M2 | 82.26879 | -0.78918 | 0.22357 | -3.52988 | 0.00042 | 0.02871 |
| OR1J4 | 94.69592 | 0.26954 | 0.07639 | 3.52869 | 0.00042 | 0.02873 |
| AMOT | 338.23579 | -0.25583 | 0.07253 | -3.52743 | 0.00042 | 0.02876 |
| RPS6KA3 | 687.09273 | -0.28461 | 0.08077 | -3.52367 | 0.00043 | 0.02907 |
| PSMA3 | 187.18292 | 0.26049 | 0.07408 | 3.51642 | 0.00044 | 0.02919 |
| ILF3 | 412.3347 | 0.16395 | 0.04661 | 3.51725 | 0.00044 | 0.02919 |
| GPC4 | 224.37032 | -0.24924 | 0.07089 | -3.51584 | 0.00044 | 0.02919 |
| DDX26B | 377.11094 | -0.26276 | 0.07462 | -3.52129 | 0.00043 | 0.02919 |
| HTR2C | 175.83852 | -0.27004 | 0.07673 | -3.51921 | 0.00043 | 0.02919 |
| MAGEB2 | 104.54339 | -0.33599 | 0.09551 | -3.51767 | 0.00044 | 0.02919 |
| POTEG | 308.88517 | -0.45205 | 0.12844 | -3.5196 | 0.00043 | 0.02919 |
| CLCN6 | 419.12924 | 0.1878 | 0.05346 | 3.5128 | 0.00044 | 0.02942 |
| ARMCX1 | 117.38886 | -0.29103 | 0.08302 | -3.50549 | 0.00046 | 0.03013 |
| RAB9A | 74.3725 | -0.32522 | 0.09288 | -3.50152 | 0.00046 | 0.03048 |
| SMIM8 | 15.18068 | 0.64205 | 0.18368 | 3.49544 | 0.00047 | 0.03091 |
| GPR119 | 113.61673 | -0.27185 | 0.07778 | -3.49489 | 0.00047 | 0.03091 |
| THOC2 | 755.50097 | -0.27726 | 0.07932 | -3.4956 | 0.00047 | 0.03091 |
| FUCA2 | 97.99063 | -0.27296 | 0.07813 | -3.49375 | 0.00048 | 0.03094 |
| PPEF1 | 329.35403 | -0.23429 | 0.06713 | -3.48994 | 0.00048 | 0.03127 |
| COLGALT2 | 313.41636 | 0.17973 | 0.05154 | 3.4875 | 0.00049 | 0.03145 |
| KRT74 | 364.47172 | 0.19814 | 0.05687 | 3.48421 | 0.00049 | 0.03173 |
| SPDYE3 | 141.84732 | 0.24642 | 0.07081 | 3.4802 | 0.0005 | 0.0321 |
| ARL17B | 24.351 | 0.52993 | 0.15232 | 3.47901 | 0.0005 | 0.03213 |
| PLXNA3 | 446.50617 | -0.27107 | 0.07794 | -3.47801 | 0.00051 | 0.03214 |
| DOK3 | 77.82812 | 0.45173 | 0.13035 | 3.46539 | 0.00053 | 0.03328 |
| TMEM150B | 68.87117 | 0.34222 | 0.09871 | 3.46681 | 0.00053 | 0.03328 |
| TTC8 | 539.4497 | 0.24666 | 0.07119 | 3.46497 | 0.00053 | 0.03328 |
| CA5B | 169.33132 | -0.24753 | 0.07143 | -3.46508 | 0.00053 | 0.03328 |
| SCRN2 | 131.3187 | 0.23948 | 0.06929 | 3.45616 | 0.00055 | 0.03413 |
| C6orf89 | 175.73012 | -0.20933 | 0.06058 | -3.45531 | 0.00055 | 0.03413 |
| SRPX2 | 430.80785 | -0.23122 | 0.06694 | -3.45414 | 0.00055 | 0.03413 |
| MSRA | 168.88571 | -0.23627 | 0.06841 | -3.45367 | 0.00055 | 0.03413 |
| ABCB7 | 627.91181 | -0.27491 | 0.07957 | -3.4551 | 0.00055 | 0.03413 |
| FAM169A | 263.40099 | -0.22012 | 0.06377 | -3.45194 | 0.00056 | 0.03424 |
| OR4D9 | 72.59531 | 0.29854 | 0.08655 | 3.44921 | 0.00056 | 0.0344 |
| ARMCX5 | 178.47575 | -0.26608 | 0.07715 | -3.44891 | 0.00056 | 0.0344 |
| HDHD1 | 111.77432 | -0.26375 | 0.07655 | -3.4456 | 0.00057 | 0.03471 |
| XCR1 | 96.54798 | 0.28308 | 0.08221 | 3.44354 | 0.00057 | 0.03486 |
| AMMECR1 | 120.30791 | -0.27406 | 0.07963 | -3.4418 | 0.00058 | 0.03497 |
| SYAP1 | 172.81036 | -0.24889 | 0.0724 | -3.43757 | 0.00059 | 0.03535 |
| ZDHHC15 | 255.09146 | -0.26158 | 0.07612 | -3.43624 | 0.00059 | 0.03535 |
| GABRQ | 270.00494 | -0.26672 | 0.07761 | -3.43657 | 0.00059 | 0.03535 |
| PCDHGB7 | 163.4294 | -0.22755 | 0.06627 | -3.43375 | 0.0006 | 0.03556 |
| NBPF11 | 46.17654 | 0.37068 | 0.10829 | 3.42303 | 0.00062 | 0.03664 |
| RBM10 | 636.28496 | -0.23381 | 0.0683 | -3.42356 | 0.00062 | 0.03664 |
| APLN | 60.26346 | -0.38157 | 0.11142 | -3.42474 | 0.00062 | 0.03664 |
| GSPT2 | 182.4416 | -0.24539 | 0.07174 | -3.42068 | 0.00062 | 0.03678 |
| IL1RAPL1 | 443.48564 | -0.29688 | 0.0868 | -3.42022 | 0.00063 | 0.03678 |
| MORC4 | 438.66365 | -0.22071 | 0.06456 | -3.41867 | 0.00063 | 0.03688 |
| NRK | 752.15504 | -0.23343 | 0.06832 | -3.41702 | 0.00063 | 0.03699 |
| TCEAL2 | 64.30961 | -0.35331 | 0.10367 | -3.40791 | 0.00065 | 0.03812 |
| ELK1 | 147.04865 | -0.33329 | 0.0979 | -3.4042 | 0.00066 | 0.0384 |
| GSTM1 | 45.70759 | -3.64304 | 1.06995 | -3.40487 | 0.00066 | 0.0384 |
| WAS | 323.8294 | -0.27495 | 0.0808 | -3.40301 | 0.00067 | 0.03845 |
| LILRB1 | 270.53951 | 0.23219 | 0.06826 | 3.4015 | 0.00067 | 0.03854 |
| HS3ST3A1 | 27.73456 | 0.50599 | 0.14883 | 3.39968 | 0.00067 | 0.03865 |
| OR5J2 | 89.98413 | 0.26451 | 0.07782 | 3.39908 | 0.00068 | 0.03865 |
| ANKRD49 | 88.39446 | -0.28699 | 0.08446 | -3.39806 | 0.00068 | 0.03867 |
| UGT2B10 | 268.2129 | 0.26158 | 0.077 | 3.39708 | 0.00068 | 0.03869 |
| ORAI2 | 78.7767 | 0.35289 | 0.1041 | 3.38976 | 0.0007 | 0.03962 |
| EMC2 | 181.79041 | -0.26359 | 0.07779 | -3.38868 | 0.0007 | 0.03966 |
| OR4D1 | 108.6855 | 0.24985 | 0.07377 | 3.38691 | 0.00071 | 0.03972 |
| GSTT2 | 29.86661 | -0.61289 | 0.18097 | -3.3866 | 0.00071 | 0.03972 |
| AWAT1 | 183.10344 | -0.22896 | 0.06767 | -3.38369 | 0.00072 | 0.04002 |
| DKC1 | 513.8922 | -0.23284 | 0.06892 | -3.37823 | 0.00073 | 0.0407 |
| KLHL41 | 125.49435 | -0.24796 | 0.07347 | -3.37519 | 0.00074 | 0.04103 |
| GOLGA8F | 50.0643 | 0.42657 | 0.12642 | 3.37432 | 0.00074 | 0.04104 |
| DDX3X | 321.32117 | -0.23103 | 0.0685 | -3.37285 | 0.00074 | 0.04113 |
| FAM86C1 | 94.23876 | 0.29669 | 0.08801 | 3.37123 | 0.00075 | 0.04125 |
| ZNF398 | 218.37476 | -0.19903 | 0.05907 | -3.36954 | 0.00075 | 0.04138 |
| USP9X | 1222.34039 | -0.2448 | 0.07275 | -3.36499 | 0.00077 | 0.04195 |
| ZNF576 | 25.36955 | 0.51694 | 0.15367 | 3.36392 | 0.00077 | 0.04199 |
| MNT | 64.90443 | 0.31825 | 0.09467 | 3.36167 | 0.00077 | 0.04221 |
| PJA1 | 205.32441 | -0.24266 | 0.07221 | -3.36023 | 0.00078 | 0.0423 |
| HSPA2 | 144.94785 | -0.24267 | 0.07223 | -3.35947 | 0.00078 | 0.0423 |
| RPGR | 838.67785 | -0.23037 | 0.06872 | -3.35214 | 0.0008 | 0.0433 |
| HIST1H2BB | 22.38595 | 0.4881 | 0.14566 | 3.351 | 0.00081 | 0.04336 |
| ABHD17A | 43.55561 | 0.39261 | 0.1172 | 3.34984 | 0.00081 | 0.04341 |
| C17orf78 | 143.98894 | -0.24624 | 0.07355 | -3.34798 | 0.00081 | 0.04358 |
| FAAH2 | 283.65056 | -0.21345 | 0.06397 | -3.33676 | 0.00085 | 0.04504 |
| LACC1 | 134.95529 | -0.24986 | 0.07489 | -3.33644 | 0.00085 | 0.04504 |
| ARX | 89.17148 | -0.33528 | 0.10049 | -3.33655 | 0.00085 | 0.04504 |
| H1FNT | 49.05727 | -0.38045 | 0.1141 | -3.33427 | 0.00086 | 0.04526 |
| FAM58A | 153.8843 | -0.24835 | 0.07453 | -3.33243 | 0.00086 | 0.04544 |
| ZC3H12A | 135.51691 | 0.24989 | 0.07505 | 3.32973 | 0.00087 | 0.04575 |
| SLC25A34 | 73.64229 | 0.3515 | 0.10566 | 3.32661 | 0.00088 | 0.04579 |
| CSTF2 | 336.85251 | -0.23 | 0.06914 | -3.32664 | 0.00088 | 0.04579 |
| BGN | 133.90353 | -0.26913 | 0.08088 | -3.3275 | 0.00088 | 0.04579 |
| PLP2 | 129.34936 | -0.27476 | 0.0826 | -3.32632 | 0.00088 | 0.04579 |
| GOLGA8A | 71.08974 | 0.35976 | 0.10839 | 3.31924 | 0.0009 | 0.04645 |
| HYAL1 | 131.23334 | 0.26806 | 0.08073 | 3.32066 | 0.0009 | 0.04645 |
| PLD3 | 161.75084 | 0.23312 | 0.0702 | 3.32075 | 0.0009 | 0.04645 |
| MIS18A | 81.12601 | -0.30383 | 0.09154 | -3.31922 | 0.0009 | 0.04645 |
| CTSD | 42.95032 | 0.39274 | 0.11855 | 3.31273 | 0.00092 | 0.0468 |
| TRIM25 | 217.52198 | -0.19883 | 0.05999 | -3.31418 | 0.00092 | 0.0468 |
| FAM122C | 246.23059 | -0.22288 | 0.06721 | -3.31625 | 0.00091 | 0.0468 |
| ATP6AP2 | 316.88173 | -0.24303 | 0.0733 | -3.31555 | 0.00091 | 0.0468 |
| CXorf65 | 107.62189 | -0.27139 | 0.08193 | -3.3125 | 0.00092 | 0.0468 |
| MMGT1 | 101.20806 | -0.28922 | 0.08731 | -3.31273 | 0.00092 | 0.0468 |
| PSRC1 | 92.11508 | 0.28022 | 0.08464 | 3.31085 | 0.00093 | 0.04683 |
| MORF4L2 | 72.17488 | -0.37345 | 0.1128 | -3.31089 | 0.00093 | 0.04683 |
| GLTPD1 | 55.34123 | 0.33212 | 0.10042 | 3.30729 | 0.00094 | 0.04692 |
| VOPP1 | 130.14989 | 0.22106 | 0.06684 | 3.3073 | 0.00094 | 0.04692 |
| MRS2 | 213.7063 | -0.21361 | 0.06457 | -3.30803 | 0.00094 | 0.04692 |
| TOMM22 | 42.55904 | -0.3899 | 0.11784 | -3.30867 | 0.00094 | 0.04692 |
| CASK | 925.60973 | -0.23192 | 0.07023 | -3.30256 | 0.00096 | 0.04759 |
| HCFC1 | 438.09755 | -0.23146 | 0.07017 | -3.29871 | 0.00097 | 0.04812 |
| CPLX1 | 32.9287 | 0.4219 | 0.12795 | 3.29745 | 0.00098 | 0.0482 |
| PPM1L | 163.07628 | -0.21052 | 0.06391 | -3.29423 | 0.00099 | 0.04863 |
| DIAPH2 | 609.29797 | -0.24175 | 0.07357 | -3.28591 | 0.00102 | 0.04996 |

**Table 5: DEGs between LMS and LM samples which have been frozen for less than 6 months with a |log2FC| value ≥ 2.**

| gene | baseMean | log2FoldC hange | IfcSE | stat | pvalue | padj |
|---|---|---|---|---|---|---|
| MT-ND1 | 31.15932 | 2.27773 | 0.33108 | 6.87971 | 0 | 0 |
| MT-CO2 | 20.2197 | 2.32409 | 0.3821 | 6.08243 | 0 | 1E-05 |
| FAM21B | 110.70743 | -3.65654 | 0.73151 | -4.9986 | 0 | 0.00034 |
| GSTM1 | 45.70759 | -3.64304 | 1.06995 | -3.40487 | 0.00066 | 0.0384 |

### (d) Comparison including samples which derive from subjects over 41 years of age

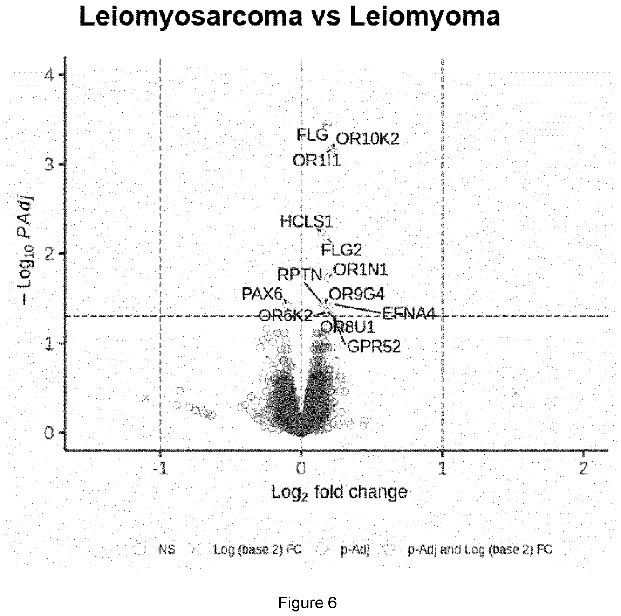

To identify DEGs in this comparison, RNAseq analysis of 57 LM and 23 LMS tumor samples which derive from subjects over 41 years of age was performed. From this analysis, a total of 13 genes were found to be significantly differentially expressed between both groups of samples, of which 12 genes were upregulated and 1 gene downregulated in LMS samples with respect to the reference value (Table 6 and Figure 6).

**Table 6: DEGs between LMS and LM samples which derive from subjects over 41 years of age.**

| gene | baseMean | log2Fold Change | IfcSE | stat | pvalue | padj |
|---|---|---|---|---|---|---|
| FLG | 1530.66941 | 0.18309 | 0.0326 | 5.61691 | 0 | 0.00035 |
| OR10K2 | 129.97238 | 0.22422 | 0.04211 | 5.32494 | 0 | 0.00068 |
| OR1I1 | 114.10074 | 0.21231 | 0.04001 | 5.30652 | 0 | 0.00068 |
| HCLS1 | 300.77477 | 0.14164 | 0.02922 | 4.84683 | 0 | 0.00572 |
| FLG2 | 1138.49528 | 0.18097 | 0.03797 | 4.76604 | 0 | 0.00686 |
| OR1N1 | 88.08035 | 0.19023 | 0.04205 | 4.52403 | 1E-05 | 0.01845 |
| EFNA4 | 87.19575 | 0.23136 | 0.05352 | 4.3231 | 2E-05 | 0.03679 |
| OR9G4 | 128.81614 | 0.17196 | 0.04034 | 4.26305 | 2E-05 | 0.03679 |
| RPTN | 361.23496 | 0.15221 | 0.03569 | 4.26449 | 2E-05 | 0.03679 |
| PAX6 | 464.04178 | -0.10252 | 0.02378 | -4.31128 | 2E-05 | 0.03679 |
| OR8U1 | 105.07897 | 0.18247 | 0.04347 | 4.19776 | 3E-05 | 0.04471 |
| OR6K2 | 135.77939 | 0.17734 | 0.04248 | 4.17477 | 3E-05 | 0.04535 |
| GPR52 | 102.87349 | 0.22309 | 0.05395 | 4.13556 | 4E-05 | 0.04969 |

### (e) Comparison including samples which have been frozen for less than 6 months and which derive from subjects over 41 years of age

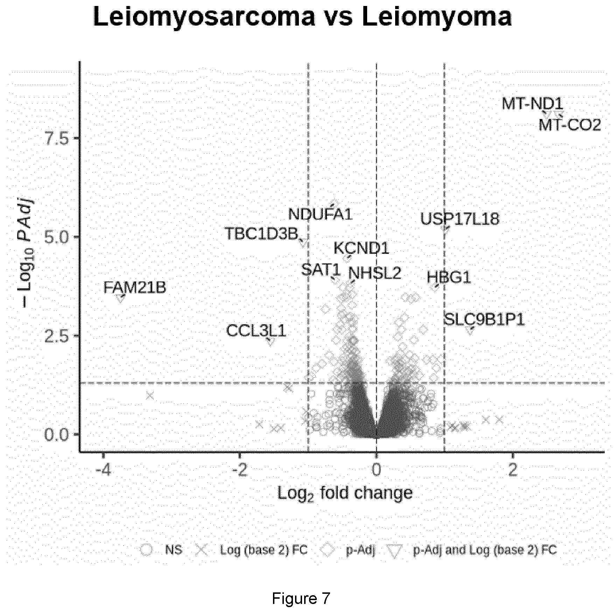

To identify DEGs in this comparison, RNAseq analysis of 16 LM and 7 LMS tumor samples which have been frozen for less than 6 months and which derive from subjects over 41 years of age was performed. From this analysis, a total of 234 genes were found to be significantly differentially expressed between both groups of samples, of which 70 genes were upregulated and 164 genes downregulated in LMS samples with respect to the reference value (Tables 7 and 8 and Figure 7).

**Table 7: DEGs between LMS and LM samples which have been frozen for less than 6 months and which derive from subjects over 41 years of age (columns are separated by commas).**

| gene | baseMean | log2FoldChange | IfcSE | stat | pvalue | padj |
|---|---|---|---|---|---|---|
| MT-CO2 | 21.5892 | 2.67223 | 0.37343 | 7.15591 | 0 | 0 |
| MT-ND1 | 33.977 | 2.4962 | 0.34665 | 7.20083 | 0 | 0 |
| NDUFA1 | 92.52969 | -0.61468 | 0.09697 | -6.33865 | 0 | 0 |
| USP17L18 | 29.7478 | 1.01398 | 0.16739 | 6.05759 | 0 | 1E-05 |
| TBC1D3B | 71.49943 | -1.07624 | 0.18239 | -5.90066 | 0 | 1E-05 |
| KCND1 | 211.83612 | -0.42998 | 0.07529 | -5.71069 | 0 | 3E-05 |
| SAT1 | 82.89351 | -0.60487 | 0.11068 | -5.465 | 0 | 0.00012 |
| NHSL2 | 340.64552 | -0.37648 | 0.06964 | -5.40648 | 0 | 0.00015 |
| HBG1 | 25.48289 | 0.85507 | 0.16011 | 5.34038 | 0 | 0.00019 |
| CCDC22 | 248.01565 | -0.38974 | 0.07349 | -5.30322 | 0 | 0.00019 |
| PIM2 | 104.4867 | -0.48957 | 0.09197 | -5.32298 | 0 | 0.00019 |
| L1CAM | 807.73309 | -0.35164 | 0.06764 | -5.19834 | 0 | 0.00029 |
| GATA1 | 189.97946 | -0.43138 | 0.08288 | -5.20518 | 0 | 0.00029 |
| KRTAP10-6 | 58.18167 | 0.57642 | 0.11188 | 5.15235 | 0 | 0.00034 |
| C16orf52 | 136.06701 | 0.4227 | 0.08262 | 5.1164 | 0 | 0.00034 |
| FLNA | 1189.51578 | -0.43732 | 0.08525 | -5.12988 | 0 | 0.00034 |
| FAM155B | 107.2491 | -0.48087 | 0.09385 | -5.12375 | 0 | 0.00034 |
| FAM21B | 122.51251 | -3.75009 | 0.7347 | -5.10424 | 0 | 0.00034 |
| PRAMEF2 | 188.76739 | -0.51838 | 0.10199 | -5.08274 | 0 | 0.00036 |
| MBD3L1 | 55.86779 | 0.50569 | 0.09979 | 5.06772 | 0 | 0.00037 |
| BCORL1 | 556.56737 | -0.35518 | 0.07117 | -4.99061 | 0 | 0.00053 |
| WDR13 | 128.12812 | -0.5121 | 0.10356 | -4.94495 | 0 | 0.00064 |
| TCEAL1 | 30.77505 | -0.74617 | 0.1515 | -4.9253 | 0 | 0.00068 |
| TSPAN7 | 99.7434 | -0.48653 | 0.09996 | -4.86742 | 0 | 0.00088 |
| CT55 | 82.13292 | -0.50917 | 0.10521 | -4.83932 | 0 | 0.00097 |
| PRPS2 | 153.31321 | -0.38251 | 0.0794 | -4.81737 | 0 | 0.00104 |
| ATP2B3 | 474.78369 | -0.39905 | 0.08318 | -4.79769 | 0 | 0.0011 |
| OR2J1 | 88.21471 | 0.41187 | 0.08598 | 4.79017 | 0 | 0.00111 |
| IQSEC2 | 566.35305 | -0.32383 | 0.06786 | -4.77181 | 0 | 0.00117 |
| SUV39H1 | 78.2061 | -0.46947 | 0.09869 | -4.75721 | 0 | 0.00122 |
| PSG3 | 191.18784 | 0.32973 | 0.07017 | 4.69894 | 0 | 0.00147 |
| STARD8 | 390.09364 | -0.34197 | 0.07276 | -4.70022 | 0 | 0.00147 |
| TEX11 | 550.37103 | -0.36849 | 0.07828 | -4.70747 | 0 | 0.00147 |
| RHOXF1 | 50.2557 | -0.52795 | 0.11314 | -4.66636 | 0 | 0.00168 |
| GRIPAP1 | 416.48632 | -0.34615 | 0.07493 | -4.6195 | 0 | 0.00204 |
| SLC9B1P1 | 8.55285 | 1.37589 | 0.30106 | 4.57011 | 0 | 0.00223 |
| RNASE13 | 23.4963 | 0.6854 | 0.14916 | 4.59517 | 0 | 0.00223 |
| PSG5 | 184.16839 | 0.36564 | 0.08005 | 4.56782 | 0 | 0.00223 |
| FAM90A1 | 375.40361 | -0.3491 | 0.0763 | -4.57519 | 0 | 0.00223 |
| PRRG3 | 93.74062 | -0.50299 | 0.10966 | -4.58677 | 0 | 0.00223 |
| TSC22D3 | 90.9068 | -0.57186 | 0.12497 | -4.57615 | 0 | 0.00223 |
| AVPR2 | 136.51613 | -0.40028 | 0.08945 | -4.47467 | 1E-05 | 0.00339 |
| PSG2 | 194.40262 | 0.31213 | 0.06987 | 4.46747 | 1E-05 | 0.00342 |
| LUZP4 | 179.20644 | -0.35078 | 0.07888 | -4.44693 | 1E-05 | 0.00368 |
| JOSD2 | 45.12215 | 0.55026 | 0.12461 | 4.41602 | 1E-05 | 0.0041 |
| PRKX | 149.84852 | -0.33716 | 0.07638 | -4.41392 | 1E-05 | 0.0041 |
| NDUFB11 | 45.77284 | -0.58868 | 0.13355 | -4.4078 | 1E-05 | 0.00413 |
| PTCHD1 | 362.03104 | -0.32562 | 0.07418 | -4.38954 | 1E-05 | 0.00428 |
| SRPX | 182.02022 | -0.34185 | 0.07816 | -4.37402 | 1E-05 | 0.00428 |
| FGD1 | 525.22522 | -0.34348 | 0.07842 | -4.38006 | 1E-05 | 0.00428 |
| HAUS1 | 141.9969 | -0.36376 | 0.08277 | -4.39503 | 1E-05 | 0.00428 |
| AWAT2 | 166.08372 | -0.42732 | 0.09769 | -4.37435 | 1E-05 | 0.00428 |
| CCL3L1 | 15.58225 | -1.55505 | 0.35507 | -4.37949 | 1E-05 | 0.00428 |
| WDR44 | 460.67254 | -0.32659 | 0.07481 | -4.36579 | 1E-05 | 0.00436 |
| FAM211A | 80.19514 | 0.38474 | 0.08969 | 4.28959 | 2E-05 | 0.00605 |
| CXorf64 | 93.96758 | -0.4248 | 0.09923 | -4.28085 | 2E-05 | 0.00618 |
| OTUD6A | 64.92447 | -0.46158 | 0.10843 | -4.25701 | 2E-05 | 0.00675 |
| DOCK11 | 1058.33572 | -0.32756 | 0.07717 | -4.24457 | 2E-05 | 0.00701 |
| RBMX2 | 116.14861 | -0.39688 | 0.09359 | -4.24084 | 2E-05 | 0.00701 |
| RNF128 | 206.84896 | -0.35228 | 0.08348 | -4.22 | 2E-05 | 0.00744 |
| SMS | 377.85047 | -0.36366 | 0.08615 | -4.22132 | 2E-05 | 0.00744 |
| TKTL1 | 291.64397 | -0.30697 | 0.07306 | -4.20144 | 3E-05 | 0.00795 |
| FUNDC1 | 85.10681 | -0.41822 | 0.09968 | -4.19555 | 3E-05 | 0.00803 |
| MAOA | 579.89228 | -0.30157 | 0.07206 | -4.18525 | 3E-05 | 0.00827 |
| GPR52 | 104.0789 | 0.34877 | 0.08381 | 4.16139 | 3E-05 | 0.00904 |
| OPA3 | 65.05525 | 0.48531 | 0.11712 | 4.14388 | 3E-05 | 0.00961 |
| CFP | 214.49641 | -0.35297 | 0.08539 | -4.13352 | 4E-05 | 0.00991 |
| CTAGE6 | 100.51088 | -0.69107 | 0.16738 | -4.12863 | 4E-05 | 0.00997 |
| OTC | 390.40795 | -0.35374 | 0.08599 | -4.11374 | 4E-05 | 0.01048 |
| GJB1 | 78.27462 | -0.48499 | 0.1186 | -4.08934 | 4E-05 | 0.01148 |
| TRIM65 | 120.41224 | 0.31463 | 0.07702 | 4.08481 | 4E-05 | 0.01154 |
| KRTAP5-10 | 38.35022 | 0.5608 | 0.13763 | 4.07454 | 5E-05 | 0.01185 |
| FOXO4 | 170.59882 | -0.31268 | 0.07678 | -4.07232 | 5E-05 | 0.01185 |
| SDHAF1 | 8.77989 | 0.95054 | 0.23438 | 4.0555 | 5E-05 | 0.01256 |
| C4orf48 | 12.80253 | 0.82875 | 0.20495 | 4.04367 | 5E-05 | 0.01287 |
| TAZ | 297.75785 | -0.32208 | 0.07965 | -4.0437 | 5E-05 | 0.01287 |
| KRTAP4-4 | 20.1293 | 0.73902 | 0.183 | 4.0383 | 5E-05 | 0.01297 |
| FKBP1A | 50.00419 | 0.42371 | 0.10499 | 4.03572 | 5E-05 | 0.01297 |
| MUC4 | 894.07082 | 0.25922 | 0.06431 | 4.03076 | 6E-05 | 0.013 |
| HSDL2 | 184.41476 | -0.25812 | 0.06411 | -4.0264 | 6E-05 | 0.013 |
| FTSJ1 | 288.54178 | -0.3415 | 0.08482 | -4.02625 | 6E-05 | 0.013 |
| ARRDC4 | 178.87937 | -0.28335 | 0.07043 | -4.02298 | 6E-05 | 0.01302 |
| PCDHA4 | 156.02938 | 0.28436 | 0.07078 | 4.01757 | 6E-05 | 0.01316 |
| NPIPB6 | 103.08118 | -0.46351 | 0.11566 | -4.00756 | 6E-05 | 0.01357 |
| FLG2 | 1160.97581 | 0.20146 | 0.05039 | 3.99807 | 6E-05 | 0.01386 |
| CHDC2 | 258.61326 | -0.3517 | 0.08805 | -3.99426 | 6E-05 | 0.01386 |
| CXorf40B | 69.5811 | -0.50163 | 0.12556 | -3.995 | 6E-05 | 0.01386 |
| OR8H2 | 89.6892 | 0.3707 | 0.0931 | 3.98188 | 7E-05 | 0.01444 |
| OCLM | 28.14701 | -0.62187 | 0.15636 | -3.97706 | 7E-05 | 0.01457 |
| NAP1L3 | 139.75768 | -0.34055 | 0.08574 | -3.97186 | 7E-05 | 0.01472 |
| FAM230A | 46.5007 | 0.52951 | 0.13369 | 3.96082 | 7E-05 | 0.01525 |
| TAGLN2 | 95.66278 | 0.33168 | 0.08401 | 3.94818 | 8E-05 | 0.0159 |
| MB21D1 | 120.36567 | -0.33532 | 0.08516 | -3.93735 | 8E-05 | 0.01629 |
| PRR23D1 | 25.43337 | -0.7959 | 0.20201 | -3.93987 | 8E-05 | 0.01629 |
| TFF2 | 65.20681 | -0.39641 | 0.101 | -3.925 | 9E-05 | 0.01696 |
| ZNF547 | 150.64797 | 0.28318 | 0.07225 | 3.91973 | 9E-05 | 0.01698 |
| HMGN5 | 120.76165 | -0.36236 | 0.0924 | -3.92154 | 9E-05 | 0.01698 |
| MNT | 63.74638 | 0.37293 | 0.09549 | 3.90536 | 9E-05 | 0.01748 |
| PNMA3 | 174.09148 | -0.33456 | 0.08561 | -3.90792 | 9E-05 | 0.01748 |
| GPKOW | 187.7711 | -0.35995 | 0.09211 | -3.90763 | 9E-05 | 0.01748 |
| ZFX | 435.26906 | -0.27401 | 0.07025 | -3.90079 | 0.0001 | 0.01752 |
| SPAG11B | 103.6872 | -0.45387 | 0.11638 | -3.90009 | 0.0001 | 0.01752 |
| GOLGA6L2 | 254.60315 | 0.31881 | 0.08196 | 3.88987 | 0.0001 | 0.01809 |
| ARMCX1 | 121.02686 | -0.35622 | 0.09166 | -3.88654 | 0.0001 | 0.01817 |
| TBC1D8B | 513.01597 | -0.34191 | 0.08804 | -3.88335 | 0.0001 | 0.01823 |
| APOO | 163.42328 | -0.35763 | 0.09222 | -3.87797 | 0.00011 | 0.01846 |
| RPL10 | 158.97733 | -0.36866 | 0.09519 | -3.87272 | 0.00011 | 0.01869 |
| CD40LG | 219.68574 | -0.34119 | 0.08817 | -3.86958 | 0.00011 | 0.01876 |
| ACE2 | 397.60777 | -0.28949 | 0.07516 | -3.85171 | 0.00012 | 0.01981 |
| SRPK3 | 172.61554 | -0.30029 | 0.07796 | -3.85174 | 0.00012 | 0.01981 |
| FAM122B | 180.01257 | -0.31221 | 0.08119 | -3.84567 | 0.00012 | 0.02012 |
| FRMPD3 | 586.82843 | -0.27857 | 0.07253 | -3.84083 | 0.00012 | 0.02034 |
| C7orf71 | 77.56832 | 0.33877 | 0.08832 | 3.8356 | 0.00013 | 0.02048 |
| OR52B6 | 94.81881 | 0.33657 | 0.08777 | 3.83477 | 0.00013 | 0.02048 |
| CHRNA10 | 106.53948 | 0.31958 | 0.08357 | 3.82392 | 0.00013 | 0.02104 |
| TMEM187 | 78.49548 | -0.4063 | 0.10625 | -3.82417 | 0.00013 | 0.02104 |
| IL17D | 9.04109 | 0.88026 | 0.23083 | 3.8134 | 0.00014 | 0.02109 |
| PIR | 212.05812 | -0.3157 | 0.08275 | -3.81501 | 0.00014 | 0.02109 |
| BEST4 | 93.94452 | -0.35238 | 0.0924 | -3.81354 | 0.00014 | 0.02109 |
| NUDT11 | 38.63433 | -0.4879 | 0.12769 | -3.82089 | 0.00013 | 0.02109 |
| OR4M2 | 87.10268 | -0.88288 | 0.23155 | -3.81292 | 0.00014 | 0.02109 |
| ORAI2 | 78.69884 | 0.37487 | 0.0984 | 3.80943 | 0.00014 | 0.02121 |
| HLA-A | 59.77343 | 0.52148 | 0.1371 | 3.80363 | 0.00014 | 0.02154 |
| SLIT1 | 34.09173 | 0.50262 | 0.13259 | 3.79076 | 0.00015 | 0.02246 |
| NO NO | 212.12092 | -0.27336 | 0.07214 | -3.78921 | 0.00015 | 0.02246 |
| B3GNT6 | 29.99957 | 0.5736 | 0.15164 | 3.78272 | 0.00016 | 0.02285 |
| MCF2 | 607.46546 | -0.28852 | 0.07631 | -3.78105 | 0.00016 | 0.02285 |
| FAM217B | 109.29185 | 0.29265 | 0.07764 | 3.76922 | 0.00016 | 0.02377 |
| CACNG5 | 127.39635 | -0.30188 | 0.08017 | -3.76564 | 0.00017 | 0.02393 |
| FAM104B | 89.65059 | -0.36164 | 0.09623 | -3.75803 | 0.00017 | 0.02448 |
| SLC16A8 | 55.04496 | 0.45658 | 0.12157 | 3.75575 | 0.00017 | 0.02451 |
| PPP3R2 | 55.05706 | -0.40123 | 0.10692 | -3.75254 | 0.00018 | 0.02464 |
| HLA-DOB | 35.53966 | 0.4728 | 0.12638 | 3.74104 | 0.00018 | 0.0256 |
| BOLL | 186.62385 | -0.27184 | 0.0729 | -3.72887 | 0.00019 | 0.02635 |
| CYLC1 | 207.1791 | -0.35462 | 0.09512 | -3.72816 | 0.00019 | 0.02635 |
| FAM3A | 164.30503 | -0.35505 | 0.09516 | -3.73102 | 0.00019 | 0.02635 |
| HIST1H2AB | 30.81724 | -0.57182 | 0.15352 | -3.72484 | 0.0002 | 0.02651 |
| IRAK1 | 210.70562 | -0.33551 | 0.09027 | -3.71689 | 0.0002 | 0.02696 |
| CBLN1 | 71.00293 | -0.36076 | 0.09706 | -3.71693 | 0.0002 | 0.02696 |
| KCNJ4 | 43.38851 | 0.45443 | 0.123 | 3.69464 | 0.00022 | 0.02784 |
| TXLNG | 204.53635 | -0.26063 | 0.07049 | -3.69739 | 0.00022 | 0.02784 |
| POTEC | 314.30446 | -0.26419 | 0.07151 | -3.69461 | 0.00022 | 0.02784 |
| POLA1 | 806.90538 | -0.26664 | 0.07213 | -3.69635 | 0.00022 | 0.02784 |
| DCTN3 | 142.04842 | -0.2855 | 0.07706 | -3.70476 | 0.00021 | 0.02784 |
| ZBTB33 | 197.51655 | -0.28815 | 0.07798 | -3.69528 | 0.00022 | 0.02784 |
| DCAF12L2 | 85.63729 | -0.37221 | 0.10072 | -3.69557 | 0.00022 | 0.02784 |
| ATOH1 | 36.27237 | -0.51322 | 0.1388 | -3.69759 | 0.00022 | 0.02784 |
| LHFPL1 | 91.86056 | -0.36723 | 0.09961 | -3.68675 | 0.00023 | 0.02852 |
| UBQLN2 | 202.9072 | -0.29233 | 0.07937 | -3.68284 | 0.00023 | 0.02876 |
| TLR8 | 301.47178 | -0.25227 | 0.06857 | -3.67897 | 0.00023 | 0.02901 |
| PLS3 | 381.98536 | -0.33765 | 0.09189 | -3.67448 | 0.00024 | 0.02914 |
| TIMM8A | 78.91066 | -0.40094 | 0.10912 | -3.67444 | 0.00024 | 0.02914 |
| DMD | 3154.41458 | -0.25957 | 0.07091 | -3.66081 | 0.00025 | 0.02977 |
| ZNF280C | 369.85679 | -0.32039 | 0.08752 | -3.66069 | 0.00025 | 0.02977 |
| MAGEH1 | 68.67607 | -0.40004 | 0.10919 | -3.66362 | 0.00025 | 0.02977 |
| DUSP9 | 62.83904 | -0.44363 | 0.12104 | -3.66512 | 0.00025 | 0.02977 |
| TMSB15A | 47.8686 | -0.47278 | 0.12908 | -3.66263 | 0.00025 | 0.02977 |
| C20orf202 | 29.056 | 0.5482 | 0.15009 | 3.65247 | 0.00026 | 0.02998 |
| LRP5L | 154.50798 | 0.32366 | 0.08859 | 3.65356 | 0.00026 | 0.02998 |
| CXorf22 | 383.2765 | -0.28656 | 0.07844 | -3.6534 | 0.00026 | 0.02998 |
| HS6ST2 | 152.87331 | -0.29351 | 0.08033 | -3.65396 | 0.00026 | 0.02998 |
| GBX1 | 85.32601 | -0.34439 | 0.09437 | -3.64933 | 0.00026 | 0.03016 |
| DOK3 | 78.16681 | 0.48883 | 0.1341 | 3.64528 | 0.00027 | 0.03026 |
| TANGO2 | 131.13535 | 0.3119 | 0.08553 | 3.64656 | 0.00027 | 0.03026 |
| MAGEC2 | 97.36354 | -0.33522 | 0.09211 | -3.63928 | 0.00027 | 0.03079 |
| RFC5 | 227.23082 | -0.21886 | 0.06021 | -3.63477 | 0.00028 | 0.03114 |
| HTATSF1 | 272.93848 | -0.27012 | 0.07438 | -3.63135 | 0.00028 | 0.03137 |
| PASD1 | 281.27358 | -0.31136 | 0.08597 | -3.62181 | 0.00029 | 0.03236 |
| HIST1H3H | 24.30736 | 0.62746 | 0.1735 | 3.61644 | 0.0003 | 0.03238 |
| GUK1 | 97.76177 | 0.33785 | 0.09344 | 3.61555 | 0.0003 | 0.03238 |
| BRCC3 | 187.71149 | -0.29599 | 0.08181 | -3.61804 | 0.0003 | 0.03238 |
| DNASE1L1 | 48.10373 | -0.50129 | 0.13861 | -3.61658 | 0.0003 | 0.03238 |
| FAM120C | 461.62994 | -0.24337 | 0.06735 | -3.61345 | 0.0003 | 0.03245 |
| FCGR3B | 48.34239 | -0.95339 | 0.26462 | -3.60293 | 0.00031 | 0.0336 |
| IDH3G | 192.6693 | -0.2897 | 0.08063 | -3.59308 | 0.00033 | 0.0347 |
| OCRL | 867.16077 | -0.24075 | 0.06707 | -3.58959 | 0.00033 | 0.03497 |
| FAM209A | 38.3982 | 0.44884 | 0.12521 | 3.5848 | 0.00034 | 0.03531 |
| UBE2NL | 58.41842 | -0.37977 | 0.10596 | -3.58412 | 0.00034 | 0.03531 |
| C3orf80 | 10.16625 | -0.98825 | 0.27653 | -3.5738 | 0.00035 | 0.03652 |
| VSIG1 | 192.79673 | -0.27325 | 0.07657 | -3.56886 | 0.00036 | 0.03701 |
| SCAND1 | 11.30085 | 0.84325 | 0.23659 | 3.56417 | 0.00037 | 0.03737 |
| CTAG2 | 54.65895 | -0.52402 | 0.14705 | -3.56346 | 0.00037 | 0.03737 |
| FAM212B | 105.97736 | 0.31501 | 0.08844 | 3.56187 | 0.00037 | 0.03739 |
| CENPM | 101.52001 | -0.30744 | 0.08636 | -3.56008 | 0.00037 | 0.03744 |
| OR10H3 | 92.73038 | 0.28073 | 0.07898 | 3.55462 | 0.00038 | 0.03786 |
| ELF4 | 355.74914 | -0.25102 | 0.07062 | -3.55427 | 0.00038 | 0.03786 |
| C1orf210 | 39.98683 | 0.42825 | 0.12058 | 3.55163 | 0.00038 | 0.03794 |
| HCN2 | 108.54973 | -0.29786 | 0.0839 | -3.55032 | 0.00038 | 0.03794 |
| RPS3A | 55.85479 | -0.37448 | 0.1055 | -3.54956 | 0.00039 | 0.03794 |
| C10orf82 | 109.5605 | -0.27682 | 0.07804 | -3.54687 | 0.00039 | 0.03812 |
| ZBED6 | 106.56348 | 0.27764 | 0.07846 | 3.53859 | 0.0004 | 0.03899 |
| PRDX4 | 172.99424 | -0.2923 | 0.08261 | -3.53822 | 0.0004 | 0.03899 |
| OR5J2 | 92.10444 | 0.2828 | 0.08013 | 3.52922 | 0.00042 | 0.03971 |
| PHKA1 | 1273.35907 | -0.27532 | 0.07799 | -3.53008 | 0.00042 | 0.03971 |
| CXorf57 | 328.54209 | -0.27572 | 0.07811 | -3.52975 | 0.00042 | 0.03971 |
| MRPL2 | 112.65458 | -0.29821 | 0.08459 | -3.52553 | 0.00042 | 0.04007 |
| LACTBL1 | 68.09389 | 0.37084 | 0.10557 | 3.51271 | 0.00044 | 0.04142 |
| CTPS2 | 381.73725 | -0.25669 | 0.07304 | -3.51441 | 0.00044 | 0.04142 |
| CXorf67 | 87.96678 | -0.37295 | 0.10616 | -3.51326 | 0.00044 | 0.04142 |
| FMR1NB | 119.13201 | -0.31315 | 0.08931 | -3.50652 | 0.00045 | 0.04204 |
| PRAMEF10 | 88.9544 | -0.63092 | 0.17995 | -3.50609 | 0.00045 | 0.04204 |
| FAM47A | 194.76758 | -0.27968 | 0.07984 | -3.50299 | 0.00046 | 0.04232 |
| ARL17B | 24.1744 | 0.56001 | 0.16 | 3.50016 | 0.00046 | 0.04256 |
| POTEG | 315.31247 | -0.44975 | 0.1287 | -3.49454 | 0.00047 | 0.04325 |
| LRRC27 | 318.08078 | -0.20142 | 0.05773 | -3.48908 | 0.00048 | 0.04393 |
| GOLGA8A | 71.64417 | 0.39538 | 0.11337 | 3.48739 | 0.00049 | 0.04399 |
| CTSD | 43.68661 | 0.42168 | 0.121 | 3.48508 | 0.00049 | 0.04412 |
| EMD | 107.46 | -0.36961 | 0.10609 | -3.48406 | 0.00049 | 0.04412 |
| CNKSR2 | 552.17294 | -0.26107 | 0.07499 | -3.48126 | 0.0005 | 0.04416 |
| ANKRD49 | 89.53959 | -0.2997 | 0.08608 | -3.48181 | 0.0005 | 0.04416 |
| DNAJC14 | 58.84576 | 0.42362 | 0.12191 | 3.47484 | 0.00051 | 0.04442 |
| HDAC8 | 365.89779 | -0.22149 | 0.06371 | -3.47646 | 0.00051 | 0.04442 |
| KLF8 | 168.37557 | -0.26003 | 0.07479 | -3.47654 | 0.00051 | 0.04442 |
| LACC1 | 136.30103 | -0.26168 | 0.07531 | -3.47459 | 0.00051 | 0.04442 |
| ATP11C | 639.03762 | -0.26821 | 0.0773 | -3.46962 | 0.00052 | 0.04504 |
| LILRB1 | 272.39712 | 0.24826 | 0.07164 | 3.4653 | 0.00053 | 0.04535 |
| CYBB | 555.10284 | -0.27093 | 0.07816 | -3.46613 | 0.00053 | 0.04535 |
| HCCS | 237.91398 | -0.27645 | 0.07988 | -3.46082 | 0.00054 | 0.0459 |
| SLC9A6 | 550.14328 | -0.27704 | 0.08012 | -3.45778 | 0.00054 | 0.046 |
| HNRNPCL1 | 84.33405 | -0.43858 | 0.12682 | -3.45827 | 0.00054 | 0.046 |
| TCEAL4 | 135.3069 | -0.27137 | 0.07858 | -3.45319 | 0.00055 | 0.04658 |
| FTH1 | 25.54703 | 0.50906 | 0.14753 | 3.45066 | 0.00056 | 0.04681 |
| OR4D1 | 110.08929 | 0.26885 | 0.07807 | 3.44376 | 0.00057 | 0.04717 |
| GLA | 328.87341 | -0.24944 | 0.07236 | -3.44711 | 0.00057 | 0.04717 |
| GDI1 | 335.4176 | -0.28865 | 0.08381 | -3.44405 | 0.00057 | 0.04717 |
| SEC16B | 20.42733 | -0.59834 | 0.1737 | -3.44465 | 0.00057 | 0.04717 |
| CLCN6 | 425.26454 | 0.20195 | 0.05872 | 3.43932 | 0.00058 | 0.04774 |
| FAM47C | 288.47746 | -0.27165 | 0.07904 | -3.43669 | 0.00059 | 0.04799 |
| LRCH2 | 319.63045 | -0.29478 | 0.08583 | -3.43434 | 0.00059 | 0.0482 |
| GK | 613.23679 | -0.29462 | 0.08587 | -3.43086 | 0.0006 | 0.04861 |
| ZC3H12A | 137.85687 | 0.26619 | 0.07772 | 3.42514 | 0.00061 | 0.04943 |
| ZNF671 | 147.30232 | 0.23868 | 0.06971 | 3.42393 | 0.00062 | 0.04943 |
| DMWD | 88.24872 | 0.33025 | 0.09653 | 3.42119 | 0.00062 | 0.04972 |
| PSMA3 | 189.47898 | 0.26857 | 0.07856 | 3.41861 | 0.00063 | 0.04998 |

**Table 8: DEGs between LMS and LM samples which have been frozen for less than 6 months and which derive from subjects over 41 years of age with a |log2FC| value ≥ 2.**

| gene | baseMean | log2FoldC hange | IfcSE | stat | pvalue | padj |
|---|---|---|---|---|---|---|
| MT-CO2 | 21.5892 | 2.67223 | 0.37343 | 7.15591 | 0 | 0 |
| MT-ND1 | 33.977 | 2.4962 | 0.34665 | 7.20083 | 0 | 0 |
| FAM21B | 122.51251 | -3.75009 | 0.7347 | -5.10424 | 0 | 0.00034 |

Overall, the results of these analyses have demonstrated that the application of this technology allows the diagnosis of the tumor in a non-invasive and objective way, based on the detection of molecular differences from circulating genetic material in peripheral blood of patients with suspected myometrial tumor (leiomyoma / leiomyosarcoma), as well as the development of biomarkers and effective therapies in the treatment of uterine leiomyosarcomas.

## Claims

1. An *in vitro* method for distinguishing between uterine leiomyosarcoma or uterine leiomyoma in a subject suspected of having one of these conditions, the method comprising:
(i) measuring the level of expression of at least one gene selected from POC1A gene, PTPRT gene, PTCHD1 gene, MUC4 gene, CENPM gene, KLHL41 gene, FLG2 gene, GPR52 gene, MB21D1 gene, MT-ND1 gene, MT-CO2 gene, FAM21B gene or GSTM1 gene in a biological sample obtained from the subject, and
(ii) comparing said level of expression with a reference value,
wherein a deviation in the level of expression of said at least one gene with respect to said reference value is indicative that the subject is affected by uterine leiomyosarcoma.

2. The method according to claim 1, wherein the deviation of the expression level with respect to the reference value in the POC1A gene, the PTPRT gene, the MUC4 gene, the FLG2 gene, the GPR52 gene, the MT-ND1 gene or the MT-CO2 gene is an increase and/or wherein the deviation of the expression level with respect to the reference value in the PTCHD1 gene, the CENPM gene, the KLHL41 gene, the MB21D1 gene, the FAM21B gene or the GSTM1 gene is a decrease.

3. An *in vitro* method for distinguishing between uterine leiomyosarcoma or uterine leiomyoma in a subject suspected of having one of these conditions, the method comprising:
(i) measuring the level of expression of at least one gene selected from the list shown in Table 2, the list shown in Table 3, the list shown in Table 4, the list shown in Table 6 or the list shown in Table 7 in a biological sample obtained from the subject, and
(ii) comparing said level of expression with a reference value,
wherein a deviation in the level of expression of said at least one gene with respect to said reference value is indicative that the subject is affected by uterine leiomyosarcoma.

4. The method according to claim 3, wherein the deviation in the level of expression of the at least one gene is a downregulation in the sample with respect to the reference value if the log2FoldChange value for said at least one gene as indicated in the Table is a negative value, or wherein the deviation in the level of expression of the at least one gene is an upregulation in the sample with respect to the reference value if the log2FoldChange value for said at least one gene as indicated in the Table is a positive value.

5. The method according to claim 1 or 3, wherein the reference value is the mean level of expression of the same gene or genes determined in a group of samples from a group of subjects with uterine leiomyoma.

6. The method according to claim 3, wherein:
(i) if the biological sample has been frozen for less than 6 months, then step (i) comprises measuring the level of expression of at least one gene selected from the list shown in Table 4,
(ii) if the biological sample derives from a subject over 41 years of age, then step (i) comprises measuring the level of expression of at least one gene selected from the list shown in Table 6, or
(iii) if the biological sample has been frozen for less than 6 months and it derives from a subject over 41 years of age, then step (i) comprises measuring the level of expression of at least one gene selected from the list shown in Table 7.

7. The method according to any one of the preceding claims, the method comprising diagnosing uterine leiomyosarcoma when the deviation in the level of expression of the at least one gene is of at least two fold with respect to the reference value, said reference value being the expression level of the same gene or genes determined in at least a sample from at least a subject with uterine leiomyoma.

8. The method according to any one of the preceding claims, wherein the determination of the expression levels of one or more genes is carried out by exome-wide gene expression from RNAseq.

9. The method according to any one of the preceding claims, wherein the biological sample is a sample containing myometrial cells or RNA derived from myometrial cells.

10. The method according to claim 9, wherein the sample containing myometrial cells or RNA derived from myometrial cells is a myometrial biopsy or a biofluid.

11. An *in vitro* method for identifying a subject suspected of having uterine leiomyosarcoma as a candidate to receive a suitable therapy to treat uterine leiomyosarcoma, the method comprising:
(i) determining whether the subject is affected by uterine leiomyosarcoma following the method of any one of claims 1 to 10; and
(ii) designating said subject as a candidate to receive a suitable therapy to treat uterine leiomyosarcoma if the subject is diagnosed as having uterine leiomyosarcoma.

12. The method according to claim 11, wherein the therapy suitable for the treatment of uterine leiomyosarcoma is selected from the group consisting of surgery, radiation therapy, chemotherapy, hormonal therapy and targeted therapy.

13. A chemotherapeutic agent, hormonal agent and/or targeted agent for use in the treatment of uterine leiomyosarcoma, wherein the subject to be treated has been identified by a method according to any of claims 1 to 10.

14. A kit, package and/or device comprising reagents adequate for implementing the methods according to any one of claims 1 to 12.

15. A computer-implemented method, wherein the method is as defined in any of claims 1 to 12.
